# EUROPEAN PATENT APPLICATION

(11) **EP 2 000 140 A1**
(43) Date of publication of application: **10.12.2008**
(21) Application number: 07011036.6
(22) Date of filing: 05.06.2007
(51) Int. Cl.: A61K 31/519, A61P 35/00

(54) **Use of derivatives of 3-oxo-2,3-dihydro-5H-thiazolo[3,2-a]pyrimidine or of 3-oxo-2,3-dihydro-5H-imidazolo[3,2-a]pyrimidine for the preparation of pharmaceutical compositions intended for the treatment of cancer**

(71) Applicant: INSTITUT NATIONAL DE LA SANTE ET DE LA RECHERCHE MEDICALE (INSERM), 75013 Paris (FR)
(72) Inventor: The designation of the inventor has not yet been filed
(74) Representative: Le Coupanec, Pascale A.M.P.

(57) **Abstract**

The present invention is related to the use of a compound of formula (I) 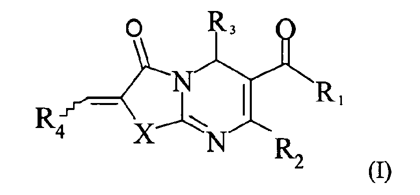 for the preparation of a pharmaceutical composition intended for the treatment of cancer and in particular for the treatment of the prostate cancer, hepatocarcinomas, head and neck cancer, oesophagus cancer, thyroid gland cancer or non-hodgkinian lymphomas and some new compounds and pharmaceutical compositions containing them.

## Description

The present invention is in the field of treatment of cancer.

The present invention more precisely deals with the use of derivatives of 3-oxo-2,3-dihydro-*5H*-thiazolo[3,2-a]pyrimidine or of 3-oxo-1,2,3-trihydro-*5H-*imidazolo[1,2-*a*]pyrimidine for the preparation of pharmaceutical compositions intended for the treatment of cancer.

The accurate transduction of intracellular signals is achieved through processes of phosphorylation-dephosphorylation of cyclin-dependent kinase (CDK) complexes which are key regulators of essential cellular events such as cell cycle regulation, growth or differentiation.

CDKs are activated by CDC25 dual-specificity phosphatases which catalyze the dephosphorylation of two adjacent phosphothreonine and phosphotyrosine residues of the CDK subunit, allowing the progression of the cell cycle into the different phases.

Three isoforms have been identified in human cells namely CDC25A, CDC25B and CDC25C. CDC25A is mainly expressed in the G1 phase and allows the progression of the cell into the S phase by dephosphorylation of the CDK2/cyclin A complex. Moreover, microinjection of CDC25A antibodies into cells induces cell arrest by blocking G1/S transition. It has also been suggested that CDC25A is involved in mitosis. CDC25B appears to play an essential role in the G2/M transition as a mitotic starter by activating the CDK1/Cyclin B complex whereas CDC25C regulates the entry into mitosis because of its ability to dephosphorylate Cdc2.

The over-expression of CDC25 is frequently associated with a wide variety of tumors of high impact and is correlated with poor prognosis. For instance, CDC25 over-expression is observed in breast, colorectal, head and neck, prostate, pancreatic, or non-small-cell lung cancers. Said target is also described as being implied in gastric-cancer, hepatocarcinomas, non-Hodgkin's lymphomas, oesophagus and thyroid gland cancers.

Because CDC25 is a therapeutically relevant target, it has been screened using different experimental approaches and several classes of inhibitors have been developed in the recent years (Lyon et al., Nat Rev Drug Discov 1:961-976, 2002, Prevost et al., Prog Cell Cycle Res 5:225-234, 2003, Brisson et al., Mol Pharmacol 66:824-833, 2004).

A few quinone derivatives have been discovered with efficient CDC25 inhibiting activity (Brezak et al., Mol Cancer Ther 4:1378-1387, 2005) (Lazo et al., J Med Chem 44:4042-4049, 2001). However, they can be subject to undesirable oxydo-reduction reactions.

Therefore, there still exists a need to develop new chemical structures showing such CDC25 inhibiting activity.

The document WO 2004/041209 discloses a method for treating microbial infections comprising the administration of a transcription factor modulating compound of formula (IV), which can pertain to the family of 3-oxo-2,3-dihydro-5*H*-thiazolo[3,2-*a*]pyrimidine derivatives.

The document EP 1 462 105 relates to inhibitors of NF-kB and methods of treating inflammatory processes in cardio-vascular diseases by use of some 5*H*-thiazolo[3,2-*a*]pyrimidine derivatives.

Moreover, document US 2004/0002526 relates to methods of treating cancer and inflammatory diseases using thiazolidinones, which modulate phospholipase D activity.

The inventors have surprisingly found that the compounds of formula (I) as defined hereinafter inhibit the enzymatic activity of CDC25. They can therefore be of particular interest in the treatment of cancer.

As a first object, the present invention deals with the use of a compound of formula (I) 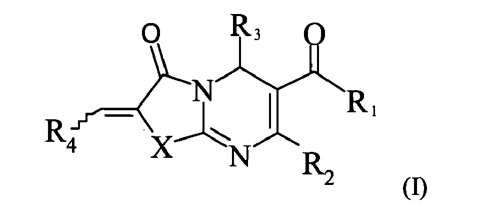 wherein
**R₁** represents a hydroxy group, an amino group, an alkyl group, an aryl group, an alkoxy group, an alkylamino group, an aralkyl group, an arylamino group, an aryloxy group, an aralkoxy group, an aralkylamino group, a -O-(CH₂)ₘ-CN group, where m represents 1, 2 or 3,
**R₂** represents an alkyl group, an optionally substituted phenyl group, a trifluoromethyl group,
**X** represents -S- or -NR₁₃-, where R₁₃ represents a hydrogen atom, an optionally substituted alkyl group, an optionally substituted alkenyl group, an optionally substituted acyl group, an aralkyl group, an aminoalkyl group, a hydroxyalkyl group, an alkoxycarbonyl group, an aralkoxycarbonyl group or a haloalkyl group,
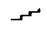 means that the exocyclic double bond may form a Z or E configuration,
**R₃** represents one of the following groups:
- (A) 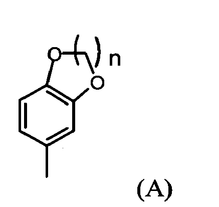 wherein **n** represents 1 or 2,
- (B) an optionally substituted phenyl group 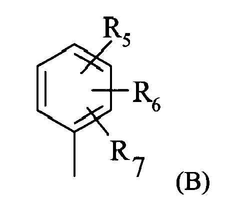 wherein **R₅**, **R₆** and **R₇** independently represent a hydrogen atom, a halogen atom, a hydroxy group, an optionally substituted alkoxy group, an optionally substituted alkyl group, an optionally substituted alkenyl group, an optionally substituted alkenyloxy group, an acyloxy group, an optionally substituted aryloxy group, an optionally substituted phenyl group, a nitro group, an amino group, an alkylaminocarbonyl group, an acylamino group, a carboxy group, a cyano group, a malonyl group, an alkoxycarbonyl group, an acyl group, an amido group, a haloalkyl group, a hydroxyalkyl group, an aminoalkyl group, a haloalkoxy group, an aralkoxycarbonyl group,
- (C) a pyridyl group 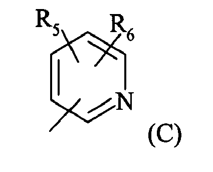 wherein **R₅** and **R₆** are as defined above,
- (D) a quinolinyl group or an isoquinolinyl group 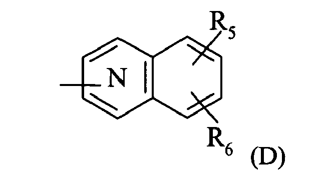 wherein **R₅** and **R₆** are as defined above,
- (E) an indolyl group, 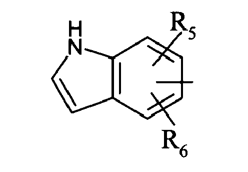 wherein **R₅** and **R₆** groups as defined above,
- (F) a naphthyl group 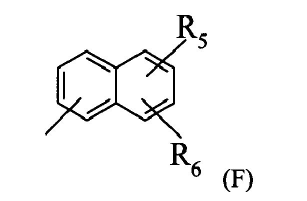 wherein **R₅** and **R₆** are as defined above,
- (G)
   a 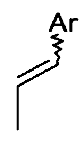 group, where Ar may be an optionally substituted phenyl group,
- (H) a thienyl group 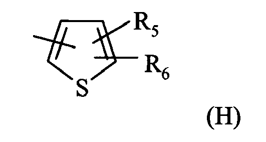 wherein **R₅** and **R₆** are as defined above and,
- (J) a furyl group 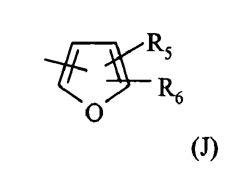 wherein **R₅** and **R₆** are as defined above,
   **R₄** represents one of the following groups:
- (α) 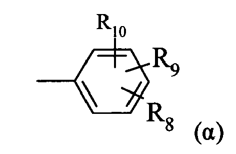 wherein **R₈, R₉** and **R₁₀** independently represent a hydrogen atom, a halogen atom, a hydroxy group, an optionally substituted alkoxy group, an optionally substituted alkyl group, an optionally substituted alkenyl group, an optionally substituted alkenyloxy group, an optionally substituted acyloxy group, an optionally substituted aryloxy group, an optionally substituted phenyl group, a nitro group, an amino group, an alkylaminocarbonyl group, an acylamino group, a carboxy group, a cyano group, a malonyl group, an alkoxycarbonyl group, an acyl group, an amido group, a haloalkyl group, a hydroxyalkyl group, an aminoalkyl group, a haloalkoxy group, an aralkoxycarbonyl group,
-(β) 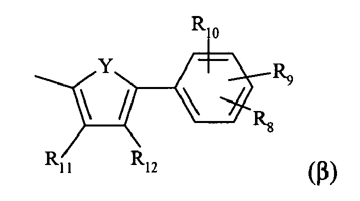 wherein
   **Y** represents -S- or -O-,
   **R₈, R₉** and **R₁₀** are as defined above, and
   **R₁₁** and **R₁₂** independently represent a hydrogen atom or a halogen atom,
- (γ) a pyridyl group 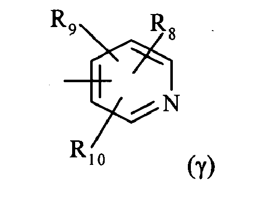 wherein **R₈, R₉** and **R₁₀** are as defined above,
- (δ) 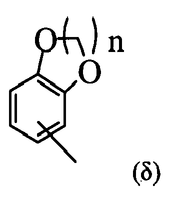 wherein **n** represents 1 or 2,
- (E)
   a 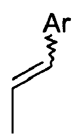 group, where **Ar** may be an optionally substituted phenyl group,
   and wherein the optionally substituted group of the alkyl group, the alkoxy group, the alkenyl group and the alkenyloxy group may be one or two groups chosen among a hydroxy group, a halogen atom, a cyano group, a carboxy group and an optionally substituted phenyl group, and
   wherein the optionally substituted group of the phenyl group, the aryloxy group and the alkenyl, alkyl, aryl or heteroaryl group of the acyl group may be one, two or three groups chosen among a nitro group, a halogen atom, a methyl group, a hydroxy group, a cyano group, a carboxy group, a methoxy group or an ethoxy group,
   for the preparation of a pharmaceutical composition intended for the treatment of cancer.

In the framework of the present invention, the substitution groups have the following meanings:
- the term "acyl" refers to an alkenyl, alkyl, aryl or heteroaryl group attached to a carbonyl group. An "acetyl" group refers to -C(O)CH₃,
- the term "acylamino" refers to an acyl group linked to an amino group,
- the term "acyloxy" refers to an acyl group attached to an oxygen atom. Included within the scope of this term are such moieties as acetoxy, n-propionoxy, isopropionoxy, n-butyroxy, valeryloxy, pivaloyloxy groups and the like,
- the term "alkenyl" as used herein refers to a straight or branched hydrocarbon radical containing one or two double bonds and containing from 2 to 10 carbon atoms. Included within the scope of this term are such moieties as ethenyl, propenyl, butenyl, 2-methylpropenyl and the like,
- the term "alkenyloxy" refers to an alkenyl group attached to an oxygen atom. Included within the scope of this term are such moieties as allyloxy,
- the term "alkoxy" refers to a saturated straight or branched-chain hydrocarbon radical containing from 1 to 10 carbon atoms attached to an oxygen atom. Included within the scope of this term are such moieties as methoxy, ethoxy, propoxy, isopropoxy, n-butoxy, isobutoxy, sec-butoxy, t-butoxy, n-pentoxy and the like,
- the term "alkoxyalkyl" refers to an alkoxy group attached to an alkyl group,
- the term "alkoxycarbonyl" refers to an alkoxy group attached to a carbonyl group. Included within the scope of this term are such moieties as methoxycarbonyl, ethoxycarbonyl, propoxycarbonyl, butoxycarbonyl and the like,
- the term "alkyl" as used herein refers to a straight or branched-chain hydrocarbon radical containing from 1 to 10 carbon atoms and cyclized manifestations thereof unless otherwise indicated. Included within the scope of this term are such moieties as methyl, ethyl, n-propyl, isopropyl cyclopropyl, n-butyl, isobutyl, s-butyl, t-butyl, t-butylmethyl, pentyl, iso-amyl, cyclopentyl, n-hexyl, cyclohexyl, cyclohexylmethyl and the like,
- the term "alkylaminocarbonyl" refers to an alkylamino group attached to a carbonyl group,
- the term "amido" refers to an amino group attached to a carbonyl group. The term "C-amido" refers to a -C(=O)-NRR' wherein R and R' are as defined hereinafter,
- the term "amino" refers to -NRR' wherein R and R' are independently hydrogen atoms, alkenyl groups, alkoxyalkyl groups, alkoxycarbonyl groups, alkyl groups, acyl groups, aryl groups or aralkyl groups,
- the term "aminoalkyl" refers to an amino group linked to an alkyl group. Included within the scope of this term are such moieties as aminomethyl, aminoethyl and the like,
- the term "aralkoxy" or "arylalkoxy" refers to an aryl group attached to an alkoxy group. Included within the scope of this term are such moieties as benzyloxy,
- the term "aralkoxycarbonyl" or "arylalkoxycarbonyl" refers to an aryloxy group attached to a carbonyl group. Included within the scope of this term are such moieties as benzyloxycarbonyl,
- the term "aralkyl" or "arylalkyl" refers to an aryl group attached to an alkyl chain. Included within the scope of this term are such moieties as benzyl,
- the term "aralkylamino" refers to an aralkyl group attached to an amino group,
- the term "aryl" refers to an optionally substituted phenyl group, naphthyl group or biphenyl group,
- the term "arylamino" refers to an aryl group attached to an amino group,
- the term "aryloxy" refers to an optionally substituted aryl group attached to an oxygen atom. Included within the scope of this term are such moieties as phenoxy group, naphthyloxy group or biphenyloxy group,
- the term "carboxy" refers to -C(=O)OH,
- the term "cyano" refers to -CN,
- the term "haloalkoxy" refers to a haloalkyl group attached to an oxygen atom,
- the term "haloalkyl" refers to an alkyl chain wherein one or more hydrogen atoms are replaced with a halogen atom. Included within the scope of this term are such moieties as fluoromethyl, difluoromethyl, trifluoromethyl, bromomethyl, dibromomethyl, tribromomethyl, chloromethyl, dichloromethyl, trichloromethyl and the like,
- the term "halogen" refers to a fluorine, a chlorine, a bromine or an iodine atom,
- the term "heteroaryl", alone or in combination, refers to a 5 to 7 membered unsaturated heterocyclic rings wherein at least one atom is a sulphur, a nitrogen or an oxygen atom. Included within the scope of this term are such moieties as furyl, thienyl, pyrrolyl, thiazolyl, oxazolyl, imidazolyl, pyridyl, pyrimidyl,
- the term "hydroxy" refers to -OH,
- the term "hydroxyalkyl" refers to a hydroxy group attached to an alkyl chain,
- the term "malonyl" refers to -CH(CO₂R)₂ wherein R is a hydrogen atom or an alkyl group.

According to one aspect, the present invention provides the use of a compound of formula (Ia) 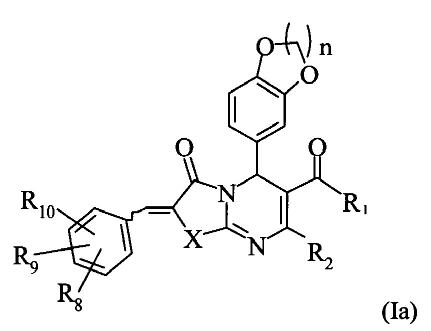 wherein R₁, R₂, R₈, R₉, R₁₀, X and n are as defined above,
for the preparation of a pharmaceutical composition intended for the treatment of cancer.

According to a preferred embodiment of this aspect, the present invention more particularly provides the use of a compound of formula (Ia) wherein
X represents -S- or -NH-,
n represents 1,
R₁ represents a hydroxy group, a benzyloxy group, an optionally substituted (C₁-C₄)alkoxy group and preferably a methoxy or an ethoxy group or an -O-(CH₂)ₘ-CN group where m represents 1, 2 or 3 and preferably where m represents 2,
R₂ represents a (C₁-C₄)alkyl group and preferably a methyl group, and
wherein R₈ and R₉ independently represent a hydrogen atom, a hydroxy group, a (C₁-C₄)alkoxy group such as a methoxy group, an -O-CH₂-CO₂H group, a (C₁-C₄)alkenyloxy group such as an allyloxy group, a carboxy group, a -CH=CH-CO₂H group, a (C₁-C₄)alkoxycarbonyl group such as a methoxycarbonyl group, a halogen atom, a nitro group or an acyloxy group such as an acetoxy group,
and R₁₀ represents a hydrogen atom, an acyloxy group such as an acetoxy group or a halogen atom,
for the preparation of a pharmaceutical composition intended for the treatment of cancer.

Among said compounds of formula (Ia), the following compounds, including their salts, may be particularly cited. The numbers given after the chemical name correspond to the numbers given in the table below:
- ethyl 5-(benzo[1,3]dioxol-5-yl)-2-(4-hydroxybenzylidene)-7-methyl-3-oxo-2,3-dihydro-5*H*-thiazolo[3,2-*α*]pyrimidine-6-carboxylate, 1
- ethyl 5-(benzo[1,3]dioxol-5-yl)-2-(4-hydroxybenzylidene)-7-methyl-3-oxo-2,3-dihydro-5*H*-thiazolo[3,2-*a*]pyrimidinium-6-carboxylate mesylate, 2 (1a)
- ethyl 5-(benzo[1,3]dioxol-5-yl)-2-benzylidene-7-methyl-3-oxo-2,3-dihydro-5*H*-thiazolo[3,2-*a*]pyrimidine-6-carboxylate, 3
- ethyl 2-(3,4-diallyloxybenzylidene)-5-(benzo[1,3]dioxol-5-yl)-7-methyl-3-oxo-2,3-dihydro-5*H*-thiazolo[3,2-*a*]pyrimidine-6-carboxylate, 4 (2a)
- benzyl 2-(4-acetoxybenzylidene)-5-(benzo[1,3]dioxol-5-yl)-7-methyl-3-oxo-2,3-dihydro-5*H*-thiazolo[3,2-*a*]pyrimidine-6-carboxylate, 5 (3a)
- 2-cyanoethyl 2-(4-acetoxybenzylidene)-5-(benzo[1,3]dioxol-5-yl)-7-methyl-3-oxo-2,3-dihydro-5*H*-thiazolo[3,2-*a*]pyrimidine-6-carboxylate, 6 (4a)
- ethyl 2-(3,4-diacetoxybenzylidene)-5-(benzo[1,3]dioxol-5-yl)-7-methyl-3-oxo-2,3-dihydro-5*H*-thiazolo[3,2-*a*]pyrimidine-6-carboxylate, 7 (5a)
- ethyl 5-(benzo[1,3]dioxol-5-yl)-2-(3,4-dimethoxybenzylidene)-7-methyl-3-oxo-2,3-dihydro-5*H*-thiazolo[3,2-*a*]pyrimidine-6-carboxylate, 8
- ethyl 2-(4-acetoxy-3,5-dibromobenzylidene)-5-(benzo[1,3]dioxol-5-yl)-7-methyl-3-oxo-2,3-dihydro-5*H*-thiazolo[3,2-*a*]pyrimidine-6-carboxylate, 9 (6a)
- ethyl 2-(2-acetoxybenzylidene)-5-(benzo[1,3]dioxol-5-yl)-7-methyl-3-oxo-2,3-dihydro-5*H*-thiazolo[3,2-*a*]pyrimidine-6-carboxylate, 10
- ethyl 2-(4-acetoxybenzylidene)-5-(benzo[1,3]dioxol-5-yl)-7-methyl-3-oxo-1,2,3-trihydro-5*H*-imidazolo[1,2-*a*]pyrimidine-6-carboxylate, 11 (7a)
- ethyl 5-(benzo[1,3]dioxol-5-yl)-2-(3,5-dibromo-4-hydroxybenzylidene)-7-methyl-3-oxo-2,3-dihydro-5*H*-thiazolo[3,2-*a*]pyrimidine-6-carboxylate, 12
- ethyl 5-(benzo[1,3]dioxol-5-yl)-2-(4-carboxyrnethoxybenzylidene)-7-methyl-3-oxo-2,3-dihydro-5*H*-thiazolo[3,2-*a*]pyrimidine-6-carboxylate, 13 (8a)
- ethyl 5-(benzo[1,3]dioxol-5-yl)-2-[4-(2-carboxyethylene)benzylidene]-7-methyl-3-oxo-2,3-dihydro-5*H*-thiazolo[3,2-*a*]pyrimidine-6-carboxylate, 14 (9a)
- ethyl 2-(3,4-diacetoxybenzylidene)-5-(benzo[1,3]dioxol-5-yl)-7-methyl-3-oxo-1,2,3-trihydro-5*H*-imidazolo[1,2-*a*]pyrimidine-6-carboxylate, 15 (10a)
- 5-(benzo[1,3]dioxol-5-yl)-2-(4-hydroxybenzylidene)-7-methyl-3-oxo-2,3-dihydro-5*H*-thiazolo[3,2-*a*]pyrimidine-6-carboxylic acid, 16 (11a)
- methyl 5-(benzo[1,3]dioxol-5-yl)-2-(4-hydroxybenzylidene)-7-methyl-3-oxo-2,3-dihydro-5*H*-thiazolo[3,2-*a*]pyrimidine-6-carboxylate, 17 (12a)
- ethyl 2-(4-acetoxybenzylidene)-5-(benzo[1,3]dioxol-5-yl)-7-methyl-3-oxo-2,3-dihydro-5*H*-thiazolo[3,2-*a*]pyrimidine-6-carboxylate, 18
- ethyl 5-(benzo[1,3]dioxol-5-yl)-7-methyl-2-(4-nitrobenzylidene)-3-oxo-2,3-dihydro-5*H*-thiazolo[3,2-*a*]pyrimidine-6-carboxylate, 19
- ethyl 2-(3-acetoxybenzylidene)-5-(benzo[1,3]dioxol-5-yl)-7-methyl-3-oxo-2,3-dihydro-5*H*-thiazolo[3,2-*a*]pyrimidine-6-carboxylate, 20 (13a)
- ethyl 5-(benzo[1,3]dioxol-5-yl)-2-(4-methoxycarbonylbenzylidene)-7-methyl-3-oxo-2,3-dihydro-5*H*-thiazolo[3,2-*a*]pyrimidine-6-carboxylate, 21
- ethyl 5-(benzo[1,3]dioxol-5-yl)-2-(3-carboxybenzylidene)-7-methyl-3-oxo-2,3-dihydro-5*H*-thiazolo[3,2-*a*]pyrimidine-6-carboxylate, 22 (14a)
- ethyl 5-(benzo[1,3]dioxol-5-yl)-2-(3-methoxycarbonylbenzylidene)-7-methyl-3-oxo-2,3-dihydro-5*H*-thiazolo[3,2-*a*]pyrimidine-6-carboxylate, 23
- 2-cyanoethyl 2-(3,4-diacetoxybenzylidehe)-5-(benzo[1,3]dioxol-5-yl)-7-methyl-3-oxo-2,3-dihydro-5*H*-thiazolo[3,2-*α*]pyrimidine-6-carboxylate, 24 (15a)
- ethyl 5-(benzo[1,3]dioxol-5-yl)-2-(3,4-dihydroxybenzylidene)-7-methyl-3-oxo-2,3-dihydro-5H-thiazolo[3,2-a]pyrimidine-6-carboxylate, 25 (16a)
- ethyl 5-(benzo[1,3]dioxol-5-yl)-2-(4-carboxybenzylidene)-7-methyl-3-oxo-2,3-dihydro-5*H*-thiazolo[3,2-*a*]pyrimidine-6-carboxylate, 26 (17a)
- ethyl 2-(3-acetoxy-4-hydroxybenzylidene)-5-(benzo[1,3]dioxol-5-yl)-7-methyl-3-oxo-2,3-dihydro-5*H*-thiazolo[3,2-*a*]pyrimidine-6-carboxylate, 27 (18a)
- ethyl 5-(benzo[1,3]dioxol-S-yl)-2-(3-hydroxy-4-methoxybenzylidene)-7-methyl-3-oxo-2,3-dihydro-5*H*-thiazolo[3,2-*a*]pyrimidine-6-carboxylate, 28

And the following compounds, and their salts, are particularly preferred:
- ethyl 2-(3,4-diacetoxybenzylidene)-5-(benzo[1,3]dioxol-5-yl)-7-methyl-3-oxo-2,3-dihydro-5*H*-thiazolo[3,2-*a*]pyrimidine-6-carboxylate, 7 (5a)
- ethyl 5-(benzo[1,3]dioxol-5-yl)-2-(4-hydroxybenzylidene)-7-methyl-3-oxo-2,3-dihydro-5*H*-thiazolo[3,2-*a*]pyrimidinium-6-carboxylate mesylate, 2 (1a)
- ethyl 5-(benzo[1,3]dioxol-5-yl)-2-(4-hydroxybenzylidene)-7-methyl-3-oxo-2,3-dihydro-5*H*-thiazolo[3,2-*a*]pyrimidine-6-carboxylate, 1
- methyl 5-(benzo[1,3]dioxol-5-yl)-2-(4-hydroxybenzylidene)-7-methyl-3-oxo-2,3-dihydro-5*H*-thiazolo[3,2-*a*]pyrimidine-6-carboxylate, 17 (12a)
- ethyl 5-(benzo[1,3]dioxol-5-yl)-2-(3,5-dibromo-4-hydroxybenzylidene)-7-methyl-3-oxo-2,3-dihydro-5*H*-thiazolo[3,2-*a*]pyrimidine-6-carboxylate, 12
- 2-cyanoethyl 2-(3,4-diacetoxybenzylidene)-5-(benzo[1,3]dioxol-5-yl)-7-methyl-3-oxo-2,3-dihydro-5*H*-thiazolo[3,2-*a*]pyrimidine-6-carboxylate, 24 (15a)

According to another aspect, the present invention provides the use of a compound of formula (Ib) 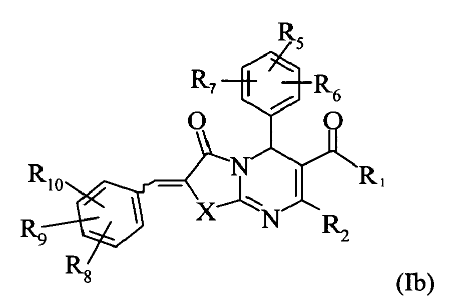 wherein R₁, R₂, R₅, R₆, R₇, R₈, R₉, R₁₀ and X are as defined above,
for the preparation of a pharmaceutical composition intended for the treatment of cancer.

According to a preferred embodiment of this aspect, the present invention more particularly provides the use of a compound of formula (Ib) wherein
X represents -S-,
R₁ represents a (C₁-C₄)alkoxy group and preferably an ethoxy group or an arylamino group and preferably a phenylamino group,
R₂ represents a (C₁-C₄)alkyl group and preferably a methyl group,
R₅, R₆ and R₇ independently represent a hydrogen atom, a (C₁-C₄)alkoxy group and preferably a methoxy group or an ethoxy group, a halogen atom, an acyloxy group such as an acetoxy group, an optionally substituted phenoxy group, a nitro group, a phenyl group or a carboxy group,
wherein the optionally substitution of the phenyl group of the phenoxy group is preferably a methoxy group or a halogen atom, and more preferably in the para position,
R₈, R₉, R₁₀ independently represent a hydrogen atom, a hydroxy group, a halogen atom, an acyloxy group, an optionally substituted (C₁-C₄)alkoxy group and preferably a methoxy group or an ethoxy group, or a carboxy group,
wherein the acyl group of the acyloxy group is an optionally substituted phenyl group, a furyl group or a methyl group,
wherein the optionally substitution of the phenyl group is a halogen atom or a (C₁-C₄)alkyl group, and
wherein the optionally substitution of the (C₁-C₄)alkoxy group is preferably a carboxy group or a halogen atom,
for the preparation of a pharmaceutical composition intended for the treatment of cancer.

According to a particular embodiment of this aspect, R₇ is a hydrogen atom or a halogen atom, preferably in the ortho position and when R₅ and R₆ are not simultaneously hydrogen atoms, at least one of R₅ and R₆ is in the meta or para position.

According to another particular embodiment of this aspect, R₈ is in the para position and represents a hydrogen atom, a hydroxy group, an acyloxy group, a methoxy group, a carboxy group, or a -O-CH₂-CO₂H group,
wherein the acyloxy group is an acetoxy group, a furylcarbonyloxy group or a phenylcarbonyloxy optionally substituted on the phenyl group by a halogen atom or a methyl group,
and R₉ and R₁₀ independently represent a hydrogen atom, a halogen atom, an acetoxy group, a hydroxy group or a (C₁-C₄)alkoxy group such as a methoxy group or an ethoxy group,

According to a further particular embodiment, R₅, R₆, R₇, R₈, R₉ and R₁₀, are simultaneously as defined in the two particular embodiments as defined above.

Among said compounds of formula (Ib), the following compounds, including their salts, may be particularly cited. The number given after the chemical name corresponds to the numbers given in the table below:
- ethyl 2-(4-acetoxybenzylidene)-5-(4-acetoxyphenyl)-7-methyl-3-oxo-2,3-dihydro-5*H*-thiazolo[3,2-*a*]pyrimidine-6-carboxylate, 29
- ethyl 2-(4-acetoxybenzylidene)-7-methyl-3-oxo-5-phenyl-2,3-dihydro-5*H-*thiazolo[3,2-*a*]pyrimidine-6-carboxylate, 30
- ethyl 2-benzylidene-7-methyl-3-oxo-5-phenyl-2,3-dihydro-5H-thiazolo[3,2-a]pyrimidine-6-carboxylate, 31
- ethyl 5-[4-(4-chlorophenoxy)phenyl]-2-(4-hydroxybenzylidene)-7-methyl-3-oxo-2,3-dihydro-5*H*-thiazolo[3,2-*a*]pyrimidine-6-carboxylate, 32 (1b)
- ethyl 2-(4-hydroxybenzylidene)-7-methyl-5-(4-nitrophenyl)-3-oxo-2,3-dihydro-5*H*-thiazolo[3,2-*a*]pyrimidine-6-carboxylate, 33 (2b)
- ethyl 2-(4-acetoxybenzylidene)-5-biphenyl-7-methyl-3-oxo-2,3-dihydro-5*H* thiazolo[3,2-*a*]pyrimidine-6-carboxylate, 34 (3b)
- ethyl 2-(4-acetoxybenzylidene)-5-[4-(4-methoxyphenoxy)phenyl]-7-methyl-3-oxo-2,3-dihydro-5*H*-thiazolo[3,2-*a*]pyrimidine-6-carboxylate, 35 (4b)
- ethyl 2-(4-acetoxybenzylidene)-5-(2-chlorophenyl)-7-methyl-3-oxo-2,3-dihydro-5*H*-thiazolo[3,2-*a*]pyrimidine-6-carboxylate, 36
- ethyl 2-(4-acetoxy-3-ethoxybenzylidene)-5-(4-chlorophenyl)-7-methyl-3-oxo-2,3-dihydro-5*H*-thiazolo[3,2-*a*]pyrimidine-6-carboxylate, 37
- ethyl 2-(3,4-dimethoxybenzylidene)-7-methyl-3-oxo-5-phenyl-2,3-dihydro-5*H*-thiazolo[3,2-*a*]pyrimidine-6-carboxylate, 38
- ethyl 2-(3,4-diacetoxybenzylidene)-5-(3,4-dimethoxyphenyl)-7-methyl-3-oxo-2,3-dihydro-5*H*-thiazolo[3,2-*a*]pyrimidine-6-carboxylate, 39 (5b)
- ethyl 2-(3,4-diacetoxybenzylidene)-5-(3,4-dichlorophenyl)-7-methyl-3-oxo-2,3-dihydro-5*H*-thiazolo[3,2-*a*]pyrimidine-6-carboxylate, 40 (6b)
- ethyl 2-(4-acetoxybenzylidene)-5-(3,4-dimethoxyphenyl)-7-methyl-3-oxo-2,3-dihydro-5*H*-thiazolo[3,2-*a*]pyrimidine-6-carboxylate, 41
- ethyl 2-(3,4-diacetoxybenzylidene)-5-(3-carboxyphenyl)-7-methyl-3-oxo-2,3-dihydro-5*H*-thiazolo[3,2-*a*]pyrimidine-6-carboxylate, 42 (7b)
- ethyl 2-(3,4-diacetoxybenzylidene)-5-(4-carboxyphenyl)-7-methyl-3-oxo-2,3-dihydro-5*H*-thiazolo[3,2-*a*]pyrimidine-6-carboxylate, 43 (8b)
- N-phenyl 2-(3,4,5-trimethoxybenzylidene)-5-(3-methoxyphenyl)-7-methyl-3-oxo-2,3-dihydro-5*H*-thiazolo[3,2-*a*]pyrimidine-6-carboxamide, 44
- ethyl 2-(2-acetoxybenzylidene)-7-methyl-3-oxo-5-phenyl-2,3-dihydro-SH thiazolo[3,2-*a*]pyrimidine-6-carboxylate, 45
- ethyl 2-(4-carboxymethoxybenzylidene)-5-(4-chlorophenyl)-7-methyl-3-oxo-2,3-dihydro-5*H*-thiazolo[3,2-*a*]pyrimidine-6-carboxylate, 46
- ethyl 2-(2-hydroxybenzylidene)-5-(3-methoxyphenyl)-7-methyl-3-oxo-2,3-dihydro-5*H*-thiazolo[3,2-*a*]pyrimidine-6-carboxylate, 47
- ethyl 2-(5-chloro-2-hydroxybenzylidene)-5-(3-methoxyphenyl)-7-methyl-3-oxo-2,3-dihydro-5*H*-thiazolo[3,2-*a*]pyrimidine-6-carboxylate, 48
- ethyl 2-(2-ethoxybenzylidene)-5-(4-ethoxyphenyl)-7-methyl-3-oxo-2,3-dihydro-5*H*-thiazolo[3,2-*a*]pyrimidine-6-carboxylate, 49
- ethyl 2-(2,5-dimethoxybenzylidene)-5-(4-methoxyphenyl)-7-methyl-3-oxo-2,3-dihydro-5*H*-thiazolo[3,2-*a*]pyrimidine-6-carboxylate, 50
- ethyl 2-(4-hydroxybenzylidene)-7-methyl-3-oxo-5-phenyl-2,3-dihydro-SH thiazolo[3,2-a]pyrimidine-6-carboxylate, 51
- ethyl 2-(4-carboxybenzylidene)-5-(4-methoxyphenyl)-7-methyl-3-oxo-2,3-dihydro-5*H*-thiazolo[3,2-*a*]pyrimidine-6-carboxylate, 52
- ethyl 5-(4-methoxyphenyl)-7-methyl-2-[4-[(3-methylbenzoyl)oxy] benzylidene]-3-oxo-2,3-dihydro-5*H*-thiazolo[3,2-*a*]pyrimidine-6-carboxylate, 53
- ethyl 2-[4-[(3-bromobenzoyl)oxy]-3-methoxybenzylidene)-5-(4-methoxyphenyl)-7-methyl-3-oxo-2,3-dihydro-5*H*-thiazolo[3,2-a]pyrimidine-6-carboxylate, 54
- ethyl 2-[3-ethoxy-4-[(fur-2-yl)oxy]benzylidene]-5-(4-methoxyphenyl)-7-methyl-3-oxo-2,3-dihydro-5*H*-thiazolo[3,2-*a*]pyrimidine-6-carboxylate, 55

And the following compounds, and their salts, are particularly preferred:
- ethyl 2-(3,4-diacetoxybenzylidene)-5-(3,4-dimethoxyphenyl)-7-methyl-3-oxo-2,3-dihydro-5*H*-thiazolo[3,2-*a*]pyrimidine-6-carboxylate, 39 (5b)

According to another aspect, the present invention provides the use of a compound of formula (Ic) 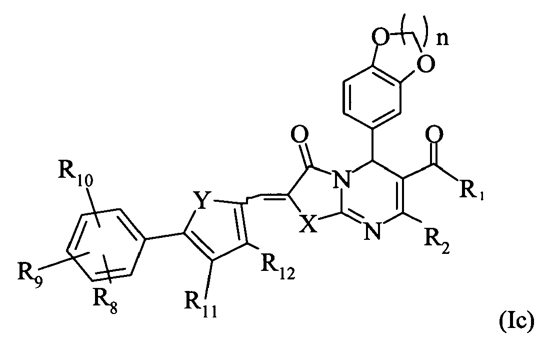 wherein R₁, R₂, R₈, R₉, R₁₀, R₁₁, R₁₂, X, Y and n are as defined above,
for the preparation of a pharmaceutical composition intended for the treatment of cancer chosen among the prostate cancer, hepatocarcinomas, head and neck cancer, oesophagus cancer, thyroid gland cancer and non hodgkinian lymphomas.

According to a preferred embodiment of this aspect, the present invention more particularly provides the use of a compound of formula (Ic) wherein
X represents -S-,
R₁ represents a (C₁-C₄)alkoxy group and preferably an ethoxy group,
R₂ represents a (C₁-C₄)alkyl group and preferably a methyl group,
n represents 1,
Y represents -O-
R₁₁ and R₁₂ represent hydrogen atoms, and
R₈, R₉ and R₁₀ independently represent a hydrogen atom or a carboxy group,
for the preparation of a pharmaceutical composition intended for the treatment of cancer chosen among the prostate cancer, hepatocarcinomas, head and neck cancer, oesophagus cancer, thyroid gland cancer and non hodgkinian lymphomas.

In the framework of this aspect, preferably only at most one of R₈, R₉ and R₁₀ is different of a hydrogen atom, which advantageously is on the meta position of the phenyl group.

According to another aspect, the present invention provides the use of a compound of formula (Id) 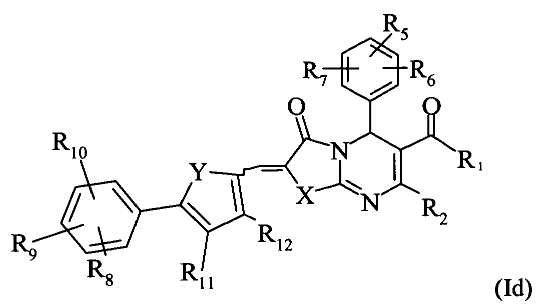 wherein R₁, R₂, R₅, R₆, R₇, R₈, R₉, R₁₀, R₁₁, R₁₂, X and Y are as defined above,
for the preparation of a pharmaceutical composition intended for the treatment of cancer chosen among the prostate cancer, hepatocarcinomas, head and neck cancer, oesophagus cancer, thyroid gland cancer and non hodgkinian lymphomas.

According to a preferred embodiment of this aspect, the present invention more particularly provides the use of a compound of formula (Id) wherein
X represents -S-,
R₁ represents a (C₁-C₄)alkoxy group and preferably an ethoxy group,
R₂ represents a (C₁-C₄)alkyl group and preferably a methyl group,
Y represents -O-
R₁ and R₁₂ represent a hydrogen atom, and
R₈, R₉ and R₁₀ independently represent a hydrogen atom or a carboxy group, and R₅, R₆, R₇ independently represent hydrogen atoms,
for the preparation of a pharmaceutical composition intended for the treatment of cancer chosen among the prostate cancer, hepatocarcinomas, head and neck cancer, oesophagus cancer, thyroid gland cancer and non hodgkinian lymphomas.

In the framework of this aspect, preferably only at most one of R₈, R₉ and R₁₀ is different of a hydrogen atom, which advantageously is on the meta position of the phenyl group.

According to another aspect, the present invention provides the use of a compound of formula (Ie) 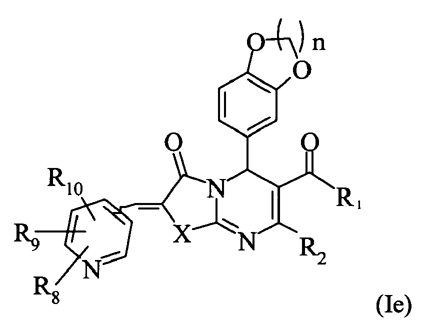 wherein R₁, R₂, R₈, R₉, R₁₀, X and n are as defined above,
for the preparation of a pharmaceutical composition intended for the treatment of cancer.

According to a preferred embodiment of this aspect, the present invention more particularly provides the use of a compound of formula (Ie) wherein
X represents -S-,
R₁ represents a (C₁-C₄)alkoxy group and preferably an ethoxy group,
R₂ represents a (C₁-C₄)alkyl group and preferably a methyl group,
n represents 1, and
R₈, R₉ and R₁₀ independently represent hydrogen atoms,
for the preparation of a pharmaceutical composition intended for the treatment of cancer.

In the framework of this aspect, preferably the nitrogen atom of the pyridyl group is on the meta position.

According to another aspect, the present invention provides the use of a compound of formula (If) 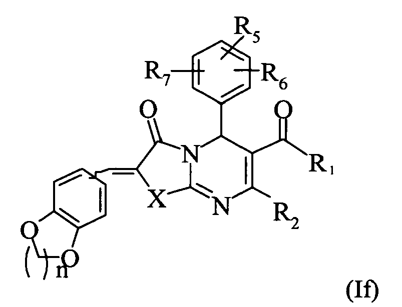 wherein R₁, R₂, R₅, R₆, R₇, X and n are as defined above,
for the preparation of a pharmaceutical composition intended for the treatment of cancer.

According to a preferred embodiment of this aspect, the present invention more particularly provides the use of a compound of formula (If) wherein
X represents -S-,
R₁ represents a (C₁-C₄)alkoxy group and preferably an ethoxy group,
R₂ represents a (C₁-C₄)alkyl group and preferably a methyl group,
n represents 1, and
R₅, R₆ and R₇ independently represent hydrogen atoms,
for the preparation of a pharmaceutical composition intended for the treatment of cancer.

According to another aspect, the present invention provides the use of a compound of formula (Ig) 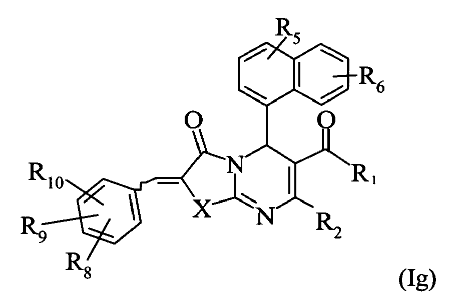 wherein R₁, R₂, R₅, R₆, R₈, R₉, R₁₀ and X are as defined above,
for the preparation of a pharmaceutical composition intended for the treatment of cancer.

According to a preferred embodiment of this aspect, the present invention more particularly provides the use of a compound of formula (Ig) wherein
X represents -S-,
R₁ represents a (C₁-C₄)alkoxy group and preferably an ethoxy group,
R₂ represents a (C₁-C₄)alkyl group and preferably a methyl group,
R₅ and R₆ represent hydrogen atoms or a (C₁-C₄)alkyl group, preferably a methyl group and preferably on the para position , and
R₈, R₉ and R₁₀ represent hydrogen atoms, hydroxy groups or acyloxy groups such as acetoxy groups,
for the preparation of a pharmaceutical composition intended for the treatment of cancer.

According to another aspect, the invention provides new compounds of formula (Ig), as defined above, with the proviso that at least two of R₈, R₉ and R₁₀ radical are acyloxy groups or with the further proviso that at least one of R₈, R₉ and R₁₀ radical is an acyloxy group with the exception of compounds substituted in the 2-position of the naphtyl group by a methoxy group.

According to another aspect, the present invention provides the use of a compound of formula (Ih) 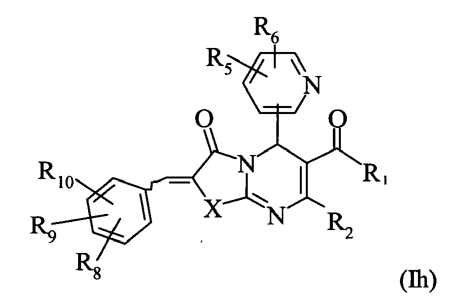 wherein R₁, R₂, R₅, R₆, R₈, R₉, R₁₀ and X are as defined above,
for the preparation of a pharmaceutical composition intended for the treatment of cancer.

According to a preferred embodiment of this aspect, the present invention more particularly provides the use of a compound of formula (Ih) wherein
X represents -S-,
R₁ represents a (C₁-C₄)alkoxy group and preferably an ethoxy group,
R₂ represents a (C₁-C₄)alkyl group and preferably a methyl group,
R₅ and R₆ represent hydrogen atoms or a phenyl group which may optionally be substituted with a (C₁-C₄)alkoxy group, and preferably a methoxy group, most preferably on the para position of the phenyl group, and
R₈, R₉ and R₁₀ independently represent hydrogen atoms or an acyloxy group and preferably an acetoxy group,
for the preparation of a pharmaceutical composition intended for the treatment of cancer.

In the framework of this aspect, preferably the nitrogen atom of the pyridyl group is on the ortho position.

Said compounds of formula (Ih) are new. Therefore, according to a further aspect, the invention provides a compound of formula (Ih) as defined above, or one of its pharmaceutically acceptable salts.

According to another aspect, the present invention provides the use of a compound of formula (Ii) 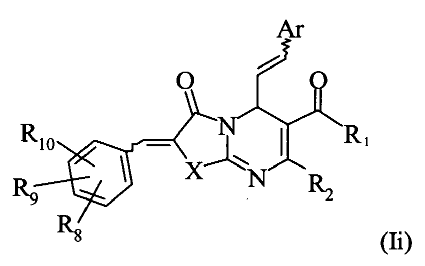 wherein
R₁, R₂, Ar, X, R₈, R₉ and R₁₀ are as defined above,
for the preparation of a pharmaceutical composition intended for the treatment of cancer.

According to a preferred embodiment of this aspect, the present invention more particularly provides the use of a compound of formula (Ii) wherein
X represents -S-,
R₁ represents a (C₁-C₄)alkoxy group and preferably an ethoxy group,
R₂ represents a (C₁-C₄)alkyl group and preferably a methyl group,
R₈, R₉ and R₁₀ independently represent hydrogen atoms or an acyloxy group preferably an acetoxy group and Ar represents an optionally substituted phenyl group,
for the preparation of a pharmaceutical composition intended for the treatment of cancer.

Some molecules represented by the general formula (I) or anyone of the formula (Ia) to (Ii) possess one asymmetric carbon atom. They can exist in the form of pure enantiomers or as a mixture of enantiomers. Moreover, the compounds according to the invention exist in the form of free bases or of addition salts with pharmaceutically acceptable acids.

According to a particular embodiment of the invention, the cancer for which the use of the compounds of formula (I) or anyone of formula (Ia) to (Ii) is intended is chosen among the prostate cancer, the colorectal cancer, hepatocarcinomas, head and neck cancer, pancreas cancer, oesophagus cancer and non hodgkinian lymphomas, thyroid gland cancer and more particularly among the prostate cancer, the colorectal cancer, hepatocarcinomas, head and neck cancer, oesophagus cancer, thyroid gland cancer and non hodgkinian lymphomas and even more particularly among prostate cancer, hepatocarcinomas, head and neck cancer, oesophagus cancer, non hodgkinian lymphomas and thyroid gland cancer.

According to an embodiment of the invention, the use according to the present invention is intended for the preparation of a pharmaceutical composition intended for the treatment of cancer not involved with the phospholipase D pathway.

Some compounds pertaining to anyone of said hereabove defined formula (Ia) to (Ii) form part of the present invention. It follows that according to an embodiment, the invention is also directed to the following new compounds:

Among compounds of formula (Ia)

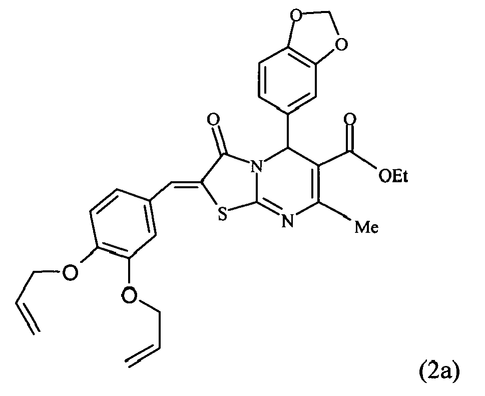

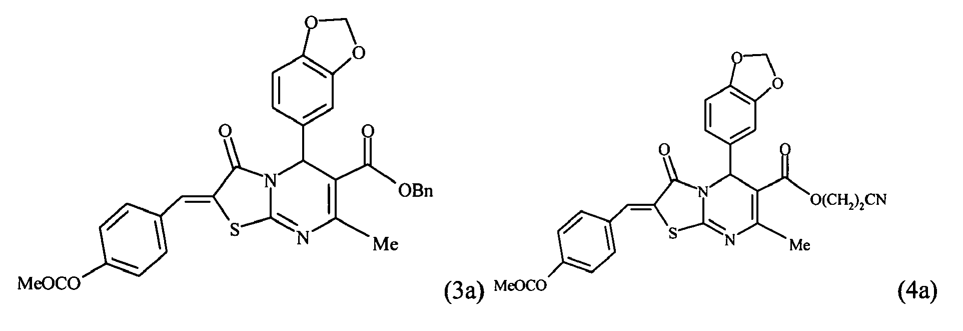

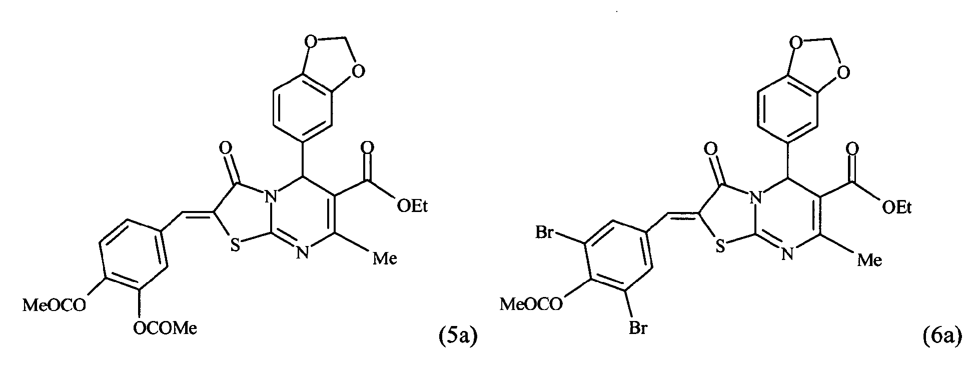

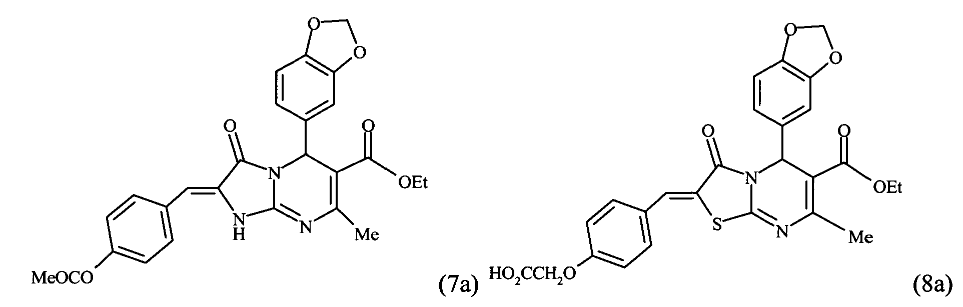

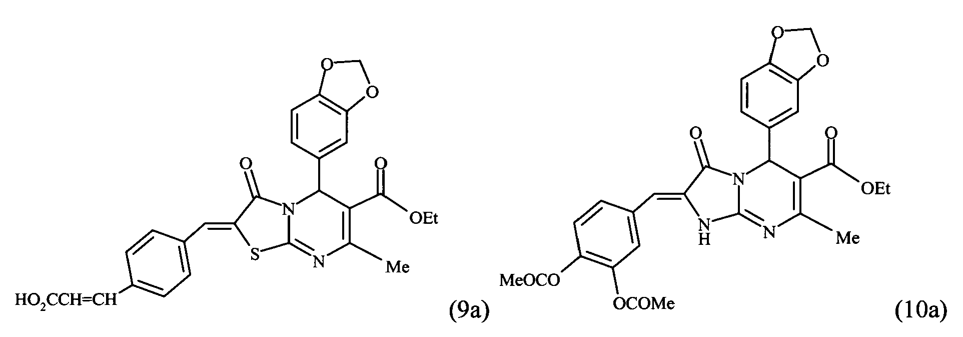

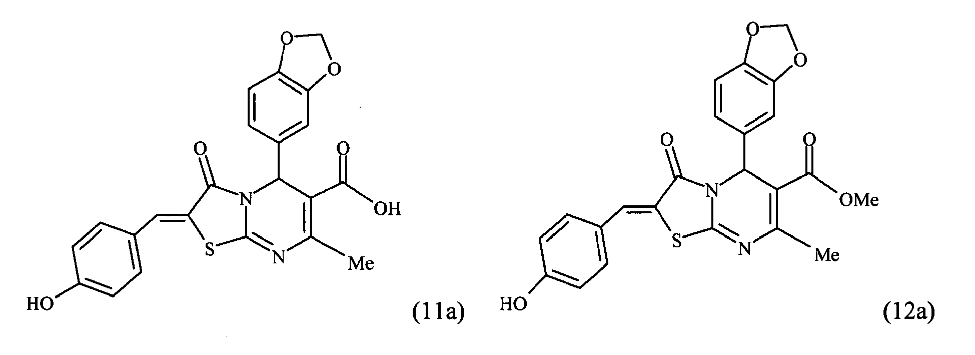

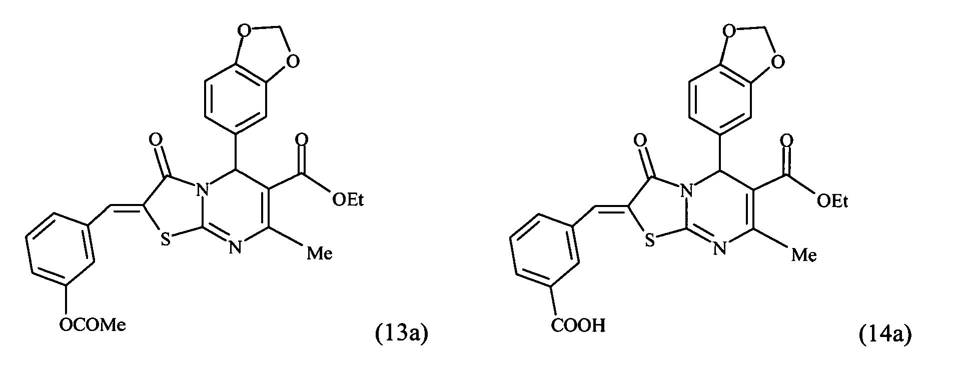

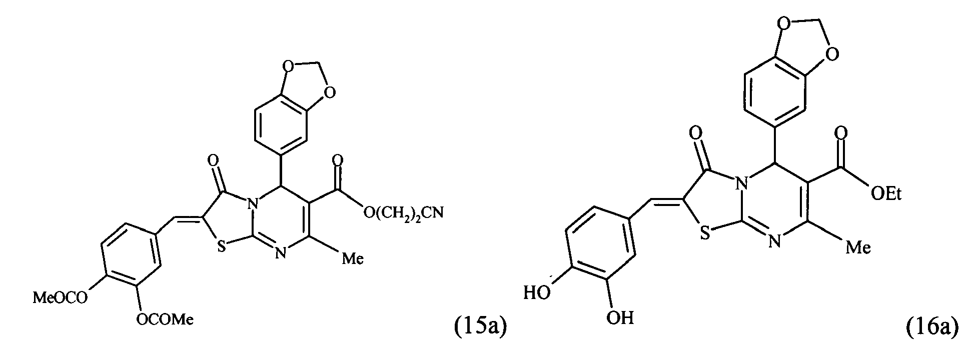

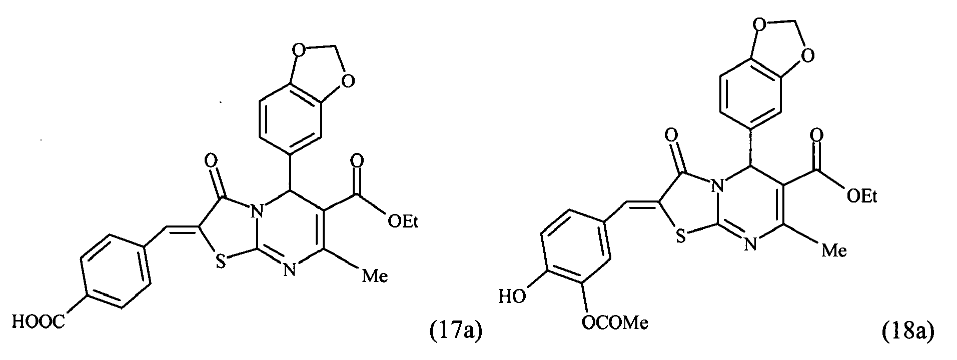

Among compounds of formula (Ib)

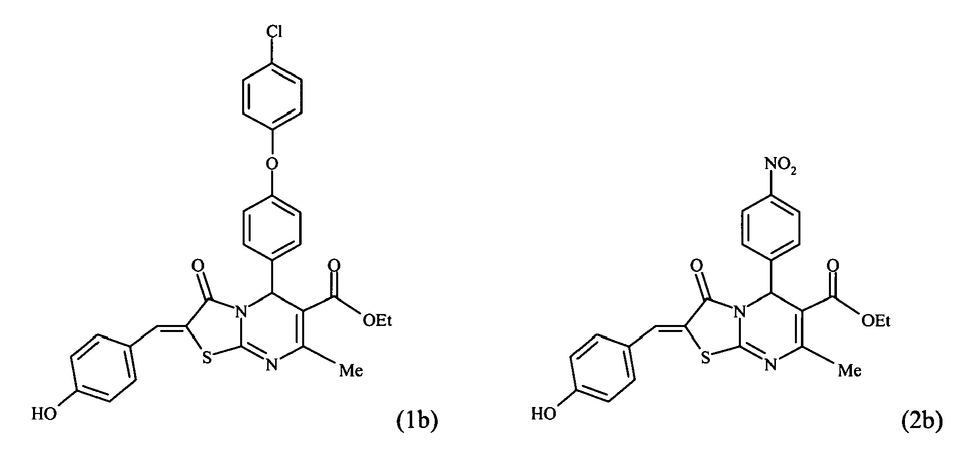

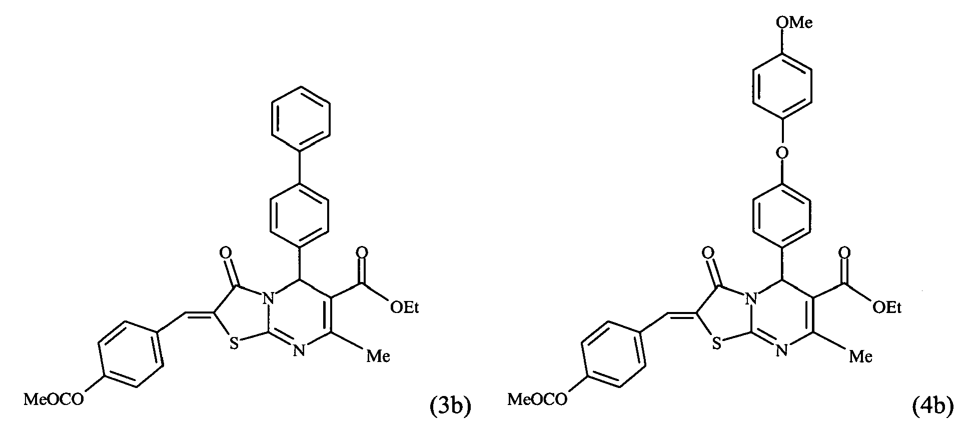

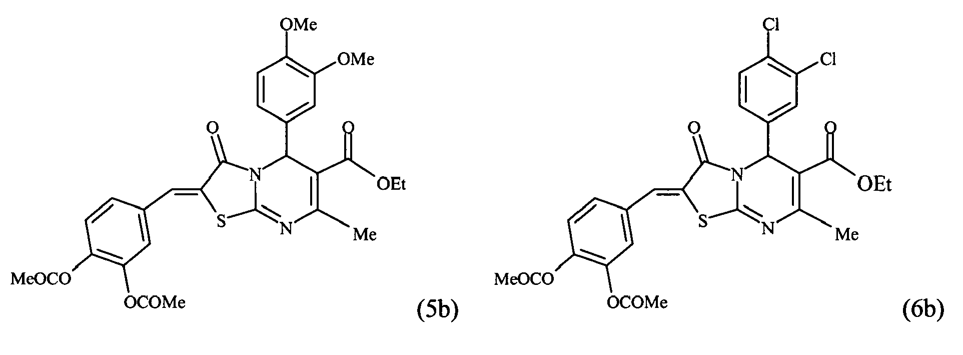

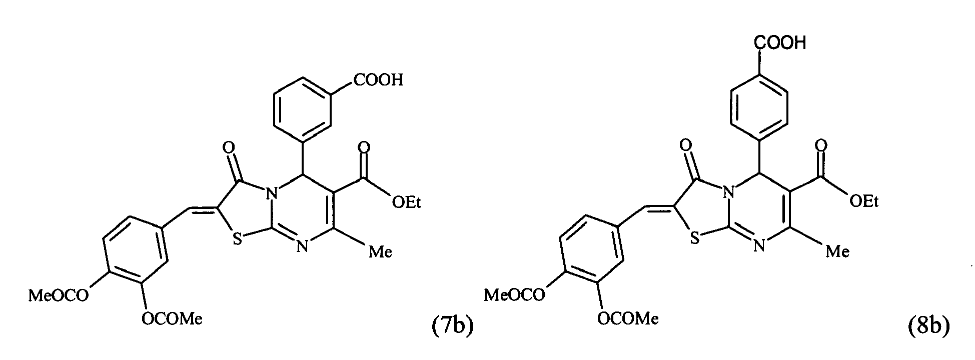

Among compounds of formula (Ic)

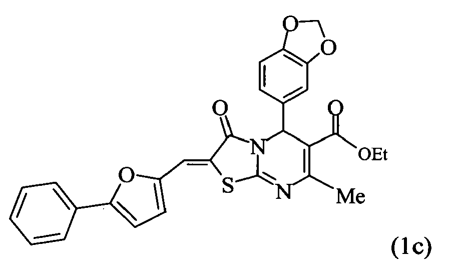

Among compounds of formula (Ig)

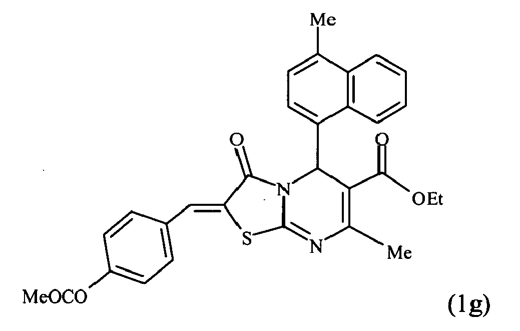

Among compounds of formula (Ih) 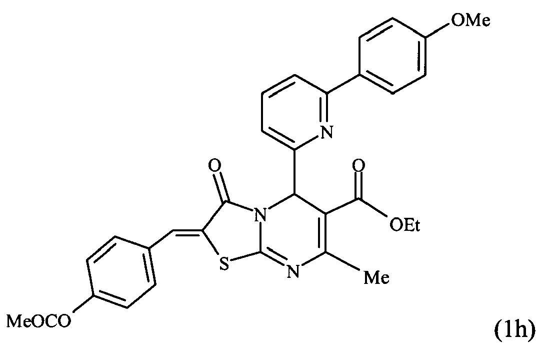 and to their pharmaceutically acceptable salts and to the following salified compound :

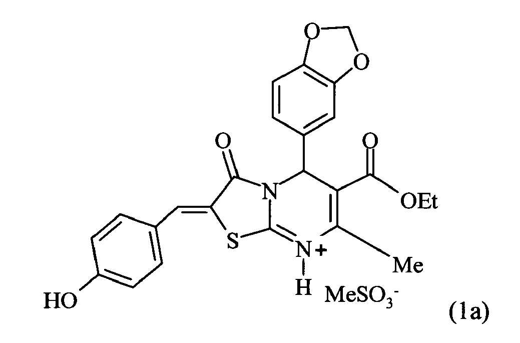

In accordance with a feature of the invention, the compounds of formula (I) in which X represents -S- are prepared according to scheme 1 below.

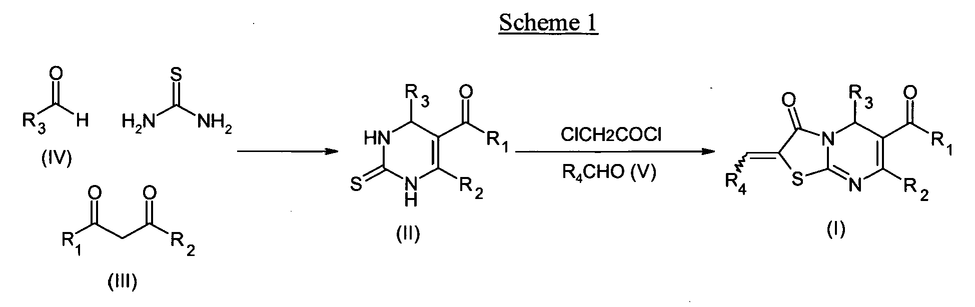

A compound of formula (IV) in which R₃ is as defined above is reacted with a compound of formula (III) in which R₁ and R₂ are as defined above and thiourea, in the presence of an acid such as *p*-toluenesulfonic acid, in a solvent such as dioxane at the boiling point of the mixture. A compound of formula (II) is obtained which is reacted with an aldehyde of formula (V) in which R₄ is as defined above in the presence of chloroacetyl chloride, of a base such as sodium acetate in acetic anhydride and acetic acid at the boiling point of the mixture to give a compound of formula (I) for example according to the method described by Tozkoporan (Tozkoporan et al., Arch Pharm Pharm Med Chem 331:201-206, 1998).

When X represents S, the compounds of general formula (I) are prepared according to a further feature of the invention according to the following scheme 2.

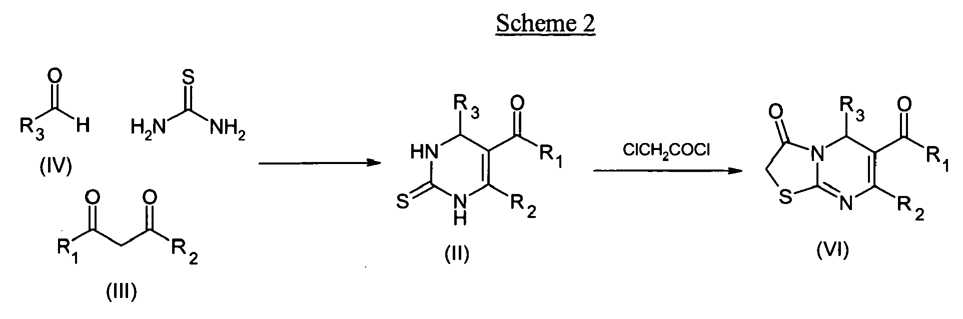

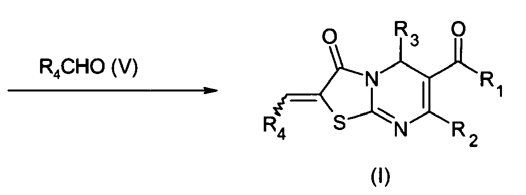

A compound of formula (IV) in which R₃ is as defined above is reacted with a compound of formula (III) in which R₁ and R₂ are as defined above and thiourea, in the presence of an acid such as p-toluenesulfonic acid, in a solvent such as dioxane at the boiling point of the mixture. A compound of formula (II) is obtained which is reacted with chloroacetyl chloride in acetic anhydride and acetic acid at the boiling point of the reaction mixture to give a compound of formula (VI). A compound of formula (VI) is finally reacted with an aldehyde of formula (V) in which R₄ is as defined above in the presence of a base such as piperidine in a solvent such as methanol to give a compound of formula (I).

When X represents NR₁₃, the compounds of general formula (I) are prepared according to a further feature of the invention according to the following scheme 3.

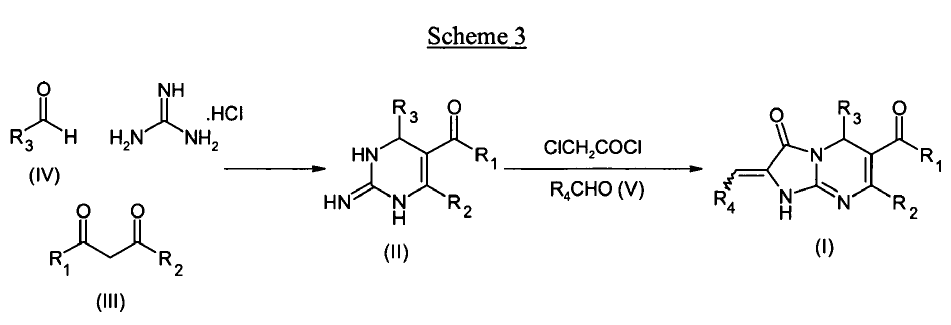

A compound of formula (IV) in which R₃ is as defined above is reacted with a compound of formula (III) in which R₁ and R₂ are as defined above and guanidine hydrochloride, in the presence of a base such as NaHCO₃, in a solvent such as dimethylformamide at a temperature of 70°C for example as described by Nilsson (Nilsson et al., J Org Chem 71:7706-7714, 2006). A compound of formula (II) is obtained which is reacted with an aldehyde of formula (V) in which R₄ is as defined above and chloroacetyl chloride in the presence of a base, such as sodium acetate in acetic anhydride and acetic acid at the boiling point of the reaction mixture to give a compound of formula (I) for example according to the method described by Tozkoporan (Tozkoporan et al., Arch Pharm Pharm Med Chem 331:201-206,1998).

The starting compounds are commercially available or are described in the literature, or can be synthesized in accordance with methods which are described therein or which are known to the person skilled in the art. In particular, compounds of formula (II) may be prepared following a Biginelli type reaction as previously described (Goss et al., J Comb Chem 8:153-155, 2006).

For example, 4-(benzo[1,3]dioxol-5-yl)-5-ethoxycarbonyl-6-methyl-3,4-dihydropyrimidine-2(*1H*)-thione was prepared as following:

A solution of 5 g (33.3 mmol) of piperonal, 3.80 g (1.5 eq) of thiourea and 1.3 g (0.2 eq) of para-toluenesulfonic acid was heated for an hour under reflux in a mixture of 100 mL of dioxan and 40 ml of isopropanol. Then, 4.9 ml (1 eq) of ethyl acetoacetate were added and the mixture was refluxed for a further 18-hour. The solution was cooled to room temperature and poured into ice-water. The precipitate was filtered to give the expected compound as a white powder with a yield of 45 % (4.8g).
mp: 89-90°C
NMR ¹H (DMSO-d₆, 250 MHz, δ), 1.12 (t, 3H, CH₃); 2.29 (s, 3H, CH₃); 4.02 (q, 2H, CH₂); 5.10 (s, 1H, CH); 6.00 (s, 2H, CH₂); 6.67 (s, 1H, aromatic H); 6.72 (d, 1H, aromatic H); 6.87 (d, 1H, aromatic H); 9.59 (s, 1H, NH); 10.31 (s, 1H, NH).

The following examples illustrate the preparation of some compounds according to the invention. The NMR spectra confirm the structures of the products obtained. The number given in brackets in the example titles correspond to those of the table which are given subsequently.

### Example 1: Compound (5a) (No. 7 of the Table)

Ethyl 2-(3,4-diacetoxybenzylidene)-5-(benzo[1,3]dioxol-5-yl)-7-methyl-3-oxo-2,3-dihydro-5*H*-thiazolo[3,2-*a*]pyrimidine-6-carboxylate.

A solution of 130 mg (0.41 mmol) of 4-(benzo[1,3]dioxol-5-yl)-5-ethoxycarbonyl-6-methyl-3,4-dihydropyrimidine-2(*1H*)-thione, 57 mg (0.41 mmol) of 3,4-dihydroxybenzaldehyde, 0.03 ml (0.41 mmol) of chloroacetyl chloride, 37 mg (0.41 mmol) of sodium acetate, 1 ml of acetic anhydride and 1.5 ml of acetic acid was refluxed for 6 hours. After cooling to room temperature, the mixture was poured into ice-water and extracted with dichloromethane. The organic layer was then washed with a solution of NaHCO₃, water and brine, dried over Na₂SO₄ and finally evaporated under reduced pressure. The residue was purified on silica gel (eluent: cyclohexane/ ethyl acetate: 90/10) and the expected product was obtained as a yellow powder with a yield of 32% (74 mg).
mp: 98°C
NMR ¹H (DMSO-d₆, 250 MHz, δ), 1.15 (t, 3H, CH₃); 2.24 (s, 6H, 2 x CH₃); 2.40 (s, 3H, CH₃); 4.07 (q, 2H, CH₂); 5.99-6.01 (m, 3H, CH, CH₂); 6.78-6.81 (s, 2H, aromatic H); 6.87-6.90 (d, 1H, aromatic H); 7.45-7.48 (d, 1H, aromatic H); 7.55-7.58 (m, 2H, aromatic H); 7.81 (s, 1H, exocyclic vinylic H).

### Example 2: Compound (13a) (No. 20 of the Table)

Ethyl 2-(3-acetoxybenzylidene)-5-(benzo[1,3]dioxol-5-yl)-7-methyl-3-oxo-2,3-dihydro-5*H*-thiazolo[3,2-*a*]pyrimidine-6-carboxylate.

A solution of 200 mg (0.62 mmol) of 4-(benzo[1,3]dioxol-5-yl)-5-ethoxycarbonyl-6-methyl-3,4-dihydropyrimidine-2(*1H*)-thione, 76 mg (0.62 mmol) of 3-hydroxybenzaldehyde, 0.05 ml (0.62 mmol) of chloroacetyl chloride, 51 mg (0.41 mmol) of sodium acetate, 1.5 ml of acetic anhydride and 2.3 ml of acetic acid was refluxed for 6 hours. After cooling to room temperature, the mixture was poured into ice-water and extracted with dichloromethane. The organic layer was then washed with a solution of NaHCO₃, water and brine, dried over Na₂SO₄ and finally evaporated under reduced pressure. The residue was purified on silica gel (eluent: cyclohexane/ethyl acetate: 90/10) and the expected product was obtained as a yellow powder with a yield of 23% (73 mg).
mp: 78°C
NMR ¹H (DMSO-d₆, 250 MHz, δ), 1.15 (t, 3H, CH₃); 2.24 (s, 3H, CH₃); 2.40 (s, 3H, CH₃); 4.05 (q, 2H, CH₂); 5.99-6.01 (m, 3H, CH, CH₂); 6.78-6.81 (s, 2H, aromatic H); 6.87-6.90 (d, 1H, aromatic H); 7.26-7.29 (d, 1H, aromatic H); 7.40 (s, 1H, aromatic H); 7.48-7.52 (d, 1 H, aromatic H); 7.51-7.63 (dd, 1H, aromatic H); 7.81 (s, 1H, exocyclic vinylic H).

### Example 3: Compound (5b) (No. 39 in the Table)

Ethyl 2-(3,4-diacetoxybenzylidene)-5-(3,4-dimethoxyphenyl)-7-methyl-3-oxo-2,3-dihydro-5*H*-thiazolo[3,2-a]pyrimidine-6-carboxylate.

A solution of 180 mg (0.55 mmol) of 5-ethoxycarbonyl-4-(3,4-dimethoxyphenyl)-6-methyl-3,4-dihydropyrimidine-2(*1H*)-thione 76 mg (0.55 mmol) of 3,4-dihydroxybenzaldehyde, 0.05 ml (0.55 mmol) of chloroacetyl chloride, 45 mg (0.55 mmol) of sodium acetate, 1 ml of acetic anhydride and 1.5 ml of acetic acid was refluxed for 6 hours. After cooling to room temperature, the mixture was poured into ice-water and extracted with dichloromethane. The organic layer was then washed with a solution of NaHCO₃, water and brine, dried over Na₂SO₄ and finally evaporated under reduced pressure. The residue was purified on silica gel (eluent: cyclohexane/ethyl acetate: 80/20) and the expected product was obtained as a yellow powder with a yield of 17% (54 mg).
mp: 118°C
NMR ¹H (DMSO-d₆, 250 MHz, δ), 1.09 (t, 3H, CH₃); 2.25 (s, 6H, 2×CH₃); 2.33 (s, 3H, CH₃); 3.66 (s, 6H, 2×OCH₃); 4.00 (q, 2H, CH₂); 5.95 (s, 1H, CH); 6.72-6.75 (d, 1H, aromatic H); 6.81-6.88 (m, 2H, aromatic H); 7.38-7.41 (d, 1H, aromatic H); 7.48-7.52 (m, 2H, aromatic H); 7.74 (s, 1H, exocyclic vinylic H).

### Example 4: Compound (16a) (No. 25 in the Table)

Ethyl 5-(benzo[1,3]dioxol-5-yl)-7-methyl-3-oxo-2,3-dihydro-5*H*-thiazolo[3,2-*a*]pyrimidine-6-carboxylate.

A solution of 484 mg (1.51 mmol) of 4-(benzo[1,3]dioxol-5-yl)-5-ethoxycarbonyl-6-methyl-3,4-dihydropyrimidine-2(1*H*)-thione, 0.12 ml (1.51 mmol) of chloroacetyl chloride in 1.8 ml of acetic anhydride and 3 ml of acetic acid was refluxed for 4 hours. After cooling, the reaction mixture was poured into water. The precipitate was then filtered and washed with water to give the expected compound as a red powder with a yield of 54% (296 mg).
NMR ¹H (DMSO-d₆, 250 MHz, δ), 1.18 (t, 3H, CH₃); 2.48 (s, 3H, CH₃); 3.80 (dd, 2H, CH₂); 4.09 (q, 2H, CH₂); 5.94 (s, 2H, CH₂); 5.99 (s, 1H, CH); 6.69 (d, 1H, aromatic H); 6.84-6.89 (m, 2H, aromatic H).

Ethyl 5-(benzo[1,3]dioxol-5-yl)-2-(3,4-dihydroxybenzylidene)-7-methyl-3-oxo-2,3-dihydro-5*H*-thiazolo[3,2-*a*]pyrimidine-6-carboxylate.

A solution of 200 mg (0.55 mmol) of ethyl 5-(benzo[1,3]dioxol-5-yl)-7-methyl-3-oxo-2,3-dihydro-5*H*-thiazolo[3,2-*a*]pyrimidine-6-carboxylate, 76 mg (0.55 mmol) of 3,4-dihydroxybenzaldehyde and 2 drops of piperidine was heated under reflux in methanol (25 ml) for 2 hours. The solvent was evaporated under reduced pressure and the residue was purified on silica gel (eluent: cyclohexane/ethyl acetate: 3/2) to give the expected product as a yellow powder with a yield of 26% (31 mg).
mp >260°C
NMR ¹H (DMSO-d₆, 250 MHz, δ), 1.16 (t, 3H, CH₃); 2.40 (s, 3H, CH₃); 4.06 (q, 2H, CH₂); 5.98-6.01 (m, 3H, CH, CH₂); 6.78-6.88 (m, 4H, aromatic H); 6.98-7.01 (m, 2H, aromatic H); 7.63 (m, 1H, exocyclic vinylic H).

### Example 5: Compound (6b) (No. 40 in the Table)

Ethyl 2-(3,4-diacetoxybenzylidene)-5-(3,4-dichlorophenyl)-7-methyl-3-oxo-2,3-dihydro-5*H*-thiazolo[3,2-*a*]pyrimidine-6-carboxylate.

A solution of 250 mg (0.72 mmol) of 4-(3,4-dichlorophenyl)-5-ethoxycarbonyl-6-methyl-3,4-dihydropyrimidine-2(*1H*)-thione, 100 mg (0.72 mmol) of 3,4-dihydroxybenzaldehyde, 0.06 ml (0.72 mmol) of chloroacetyl chloride, 59 mg (0.72 mmol) of sodium acetate, 1 ml of acetic anhydride and 1.5 ml of acetic acid was refluxed for 6 hours. After cooling to room temperature, the mixture was poured into ice-water and extracted with dichloromethane. The organic layer was then washed with a solution of NaHCO₃, water and brine, dried over Na₂SO₄ and finally evaporated under reduced pressure. The residue was purified on silica gel (eluent: cyclohexane/ethyl acetate: 95/5) and the expected product was obtained as a yellow powder with a yield of 34% (144 mg).
mp: 95°C
NMR ¹H (CDCl₃, 250 MHz, δ), 1.23 (t, 3H, CH₃); 2.33 (s, 6H, 2×CH₃); 2.54 (s, 3H, CH₃); 4.15 (q, 2H, CH₂); 6.15 (s, 1H, CH); 7.25-7.42 (m, 5H, aromatic H); 6.69 (s, 1H, aromatic H); 7.88 (s, 1H, exocyclic vinylic H).

The following table illustrates the chemical structures and the physical properties of some compounds according to the invention.

**TABLE**

| | | | | | | | | | |
|---|---|---|---|---|---|---|---|---|---|
| 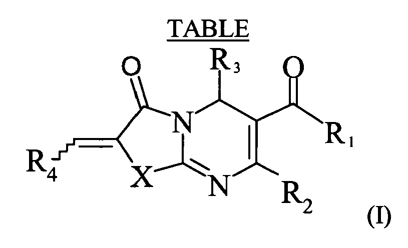 | | | | | | | | | |

| **Compounds of formula (Ia)** | | | | | | | | | |
|---|---|---|---|---|---|---|---|---|---|
| 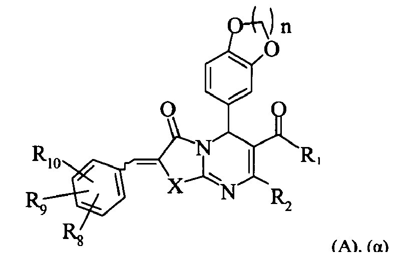 | | | | | | | | | |

| **No (compound number)** | **X** | **R₁** | **R₂** | **R₈** | **R₉** | **R₁₀** | **n** | **salt** | **Melting point (°C)** |
|---|---|---|---|---|---|---|---|---|---|
| 1 | S | OEt | Me | p-OH | H | H | 1 | - | >260°C |
| 2 (1a) | S | OEt | Me | p-OH | H | H | 1 | MeSO₃H | 243°C |
| 3 | S | OEt | Me | H | H | H | 1 | - | 185°C |
| 4 (2a) | S | OEt | Me | p-OAllyl | m-OAllyl | H | 1 | - | 147°C |
| 5 (3a) | S | OBn | Me | p-OCOMe | H | H | 1 | - | 143°C |
| 6 (4a) | S | O(CH₂)₂CN | Me | p-OCOMe | H | H | 1 | - | 155°C |
| 7 (5a) | S | OEt | Me | p-OCOMe | m-OCOMe | H | 1 | - | 98°C |
| 8 | S | OEt | Me | p-OMe | m-OMe | H | 1 | - | 177°C |
| 9 (6a) | S | OEt | Me | p-OCOMe | m-Br | m-Br | 1 | - | 144°C |
| 10 | S | OEt | Me | H | H | o-OCOMe | 1 | - | 178°C |
| 11 (7a) | NH | OEt | Me | p-OCOMe | H | H | 1 | - | 240°C |
| 12 | S | OEt | Me | p-OH | m-Br | m-Br | 1 | - | 219°C |
| 13 (8a) | S | OEt | Me | p-OCH₂CO₂H | H | H | 1 | - | 124°C |
| 14 (9a) | S | OEt | Me | p-CH=CHCO₂H | H | H | 1 | - | >260°C |
| 15 (10a) | NH | OEt | Me | p-OCOMe | m-OCOMe | H | 1 | - | 134°C |
| 16 (11a) | S | OH | Me | p-OH | H | H | 1 | - | 182°C |
| 17 (12a) | S | OMe | Me | p-OH | H | H | 1 | - | >260°C |
| 18 | S | OEt | Me | p-OCOMe | H | H | 1 | - | >260°C |
| 19 | S | OEt | Me | p-NO₂ | H | H | 1 | - | 150°C |
| 20 (13a) | S | OEt | Me | H | m-OCOMe | H | 1 | - | 78°C |
| 21 | S | OEt | Me | p-CO₂Me | H | H | 1 | - | 149°C |
| 22 (14a) | S | OEt | Me | H | m-CO₂H | H | 1 | - | 255°C |
| 23 | S | OEt | Me | H | m-CO₂Me | H | 1 | - | 170°C |
| 24 (15a) | S | O(CH₂)₂CN | Me | p-OCOMe | m-OCOMe | H | 1 | - | 116°C |
| 25 (16a) | S | OEt | Me | p-OH | m-OH | H | 1 | - | >260°C |
| 26 (17a) | S | OEt | Me | p-COOH | H | H | 1 | - | 235°C |
| 27 (18a) | S | OEt | Me | p-OH | m-OCOMe | H | 1 | - | 141°C |
| 28 | S | OEt | Me | p-OMe | m-OH | H | 1 | - | 227°C |

| **Compounds of formula (Ib)** | | | | | | | | | | | |
|---|---|---|---|---|---|---|---|---|---|---|---|
| 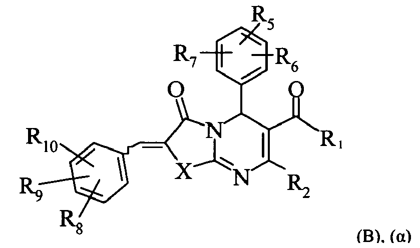 | | | | | | | | | | | |

| **No (compound number)** | **X** | **R₁** | **R₂** | **R₈** | **R₉** | **R₁₀** | **R₅** | **R₆** | **R₇** | **.Salt** | **Melting point (°C)** |
|---|---|---|---|---|---|---|---|---|---|---|---|
| 29 | S | OEt | Me | p-OCOMe | H | H | p-OCOMe | H | H | - | 202°C |
| 30 | S | OEt | Me | p-OCOMe | H | H | H | H | H | - | 191°C |
| 31 | S | OEt | Me | H | H | H | H | H | H | - | 143-144°C |
| 32 (1b) | S | OEt | Me | p-OH | H | H | p-(p-ClPhO) | H | H | - | 238°C |
| 33 (2b) | S | OEt | Me | p-OH | H | H | p-NO₂ | H | H | - | 254°C |
| 34 (3b | S | OEt | Me | p-OCOMe | H | H | p-Ph | H | H | - | n.d. |
| 35 (4b) | S | OEt | Me | p-OCOMe | H | H | p-(p-OMePhO) | H | H | - | 154°C |
| 36 | S | OEt | Me | p-OCOMe | H | H | H | H | o-Cl | - | 160°C |
| 37 | S | OEt | Me | p-OCOMe | m-OEt | H | p-Cl | H | H | - | 176°C |
| 38 | S | OEt | Me | p-OMe | m-OMe | H | H | H | H | - | 160°C |
| 39 (5b) | S | OEt | Me | p-OCOMe | m-OCOMe | H | p-OMe | m-OMe | H | - | 118°C |
| 40 (6b) | S | OEt | Me | p-OCOMe | m-OCOMe | H | p-Cl | m-Cl | H | - | 95°C |
| 41 | S | OEt | Me | p-OCOMe | H | H | p-OMe | m-OMe | H | - | 173°C |
| 42 (7b) | S | OEt | Me | p-OCOMe | m-OCOMe | H | H | m-COOH | H | - | 174°C |
| 43 (8b) | S | OEt | Me | p-OCOMe | m-OCOMe | H | p-COOH | H | H | - | 175°C |
| 44 | S | NH Ph | Me | p-OMe | m-OMe | m-OMe | H | m-OMe | H | - | n.d. |
| 45 | S | OEt | Me | H | H | o-OCOMe | H | H | H | - | n.d. |
| 46 | S | OEt | Me | p-OCH₂CO₂H | H | H | p-Cl | H | H | - | n.d. |
| 47 | S | OEt | Me | H | H | o-OH | H | m-OMe | H | - | n.d. |
| 48 | S | OEt | Me | H | 5-Cl | 2-OH | H | m-OMe | H | - | n.d. |
| 49 | S | OEt | Me | H | H | o-OEt | p-OEt | H | H | - | n.d. |
| 50 | S | OEt | Me | H | 5-OMe | 2-OMe | p-OMe | H | H | - | n.d. |
| 51 | S | OEt | Me | p-OH | H | H | H | H | H | - | 257°C |
| 52 | S | OEt | Me | p-CO₂H | H | H | p-OMe | H | H | - | 251°C |
| 53 | S | OEt | Me | p-OC(O)-(m-Me)Ph | H | H | p-OMe | H | H | - | 193°C |
| 54 | S | OEt | Me | p-OC(O)-(m-Br)Ph | m-OMe | H | p-OMe | H | H | - | 168°C |
| 55 | S | OEt | Me | p-OC(O)-fur-2-yl | m-OEt | H | p-OMe | H | H | - | 197°C |

| **Compounds of formula (Ic)** | | | | | | | | | | | | |
|---|---|---|---|---|---|---|---|---|---|---|---|---|
| 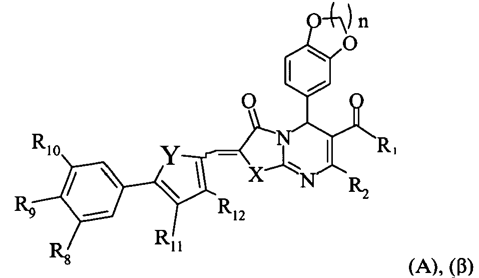 | | | | | | | | | | | | |

| **No (compound number)** | **X** | **R₁** | **R₂** | **R₈** | **R₉** | **R₁₀** | **R₁₁** | **R₁₂** | **Y** | **n** | **Salt** | **Melting point (°C)** |
|---|---|---|---|---|---|---|---|---|---|---|---|---|
| 56 (1c) | S | OEt | Me | H | H | H | H | H | O | 1 | - | 196-197°C |
| 57 | S | OEt | Me | COOH | H | H | H | H | O | 1 | - | 176°C |

| **Compounds of formula (Id)** | | | | | | | | | | | | | | |
|---|---|---|---|---|---|---|---|---|---|---|---|---|---|---|
| 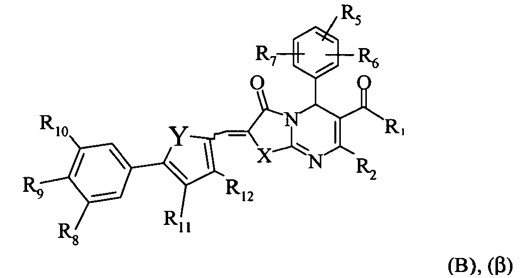 | | | | | | | | | | | | | | |

| **No (compound number)** | **X** | **R₁** | **R₂** | **R₈** | **R₉** | **R₁₀** | **R₁₁** | **R₁₂** | **Y** | **R₅** | **R₆** | **R₇** | **Salt** | **Melting point (°C)** |
|---|---|---|---|---|---|---|---|---|---|---|---|---|---|---|
| 58 | S | OEt | Me | H | H | H | H | H | O | H | H | H | - | 233°C |
| 59 | S | OEt | Me | COOH | H | H | H | H | O | H | H | H | - | 176°C |

| **Compounds of formula (Ie)** | | | | | | | | | |
|---|---|---|---|---|---|---|---|---|---|
| 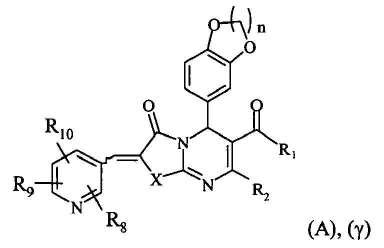 | | | | | | | | | |

| **No (compound number)** | **X** | **R₁** | **R₂** | **R₈** | **R₉** | **R₁₀** | **n** | **Salt** | **Melting point (°C)** |
|---|---|---|---|---|---|---|---|---|---|
| 60 | S | OEt | Me | H | H | H | 1 | - | 198°C |

| **Compounds of formula (If)** | | | | | | | | | |
|---|---|---|---|---|---|---|---|---|---|
| 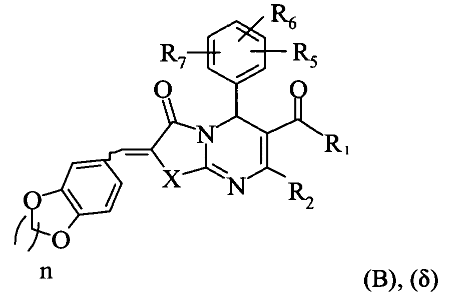 | | | | | | | | | |

| **No (compound number)** | **X** | **R₁** | **R₂** | **n** | **R₅** | **R₆** | **R₇** | **Salt** | **Melting point (°C)** |
|---|---|---|---|---|---|---|---|---|---|
| 61 | S | OEt | Me | 1 | H | H | H | - | 128°C |

| **Compounds of formula (Ig)** | | | | | | | | | | |
|---|---|---|---|---|---|---|---|---|---|---|
| 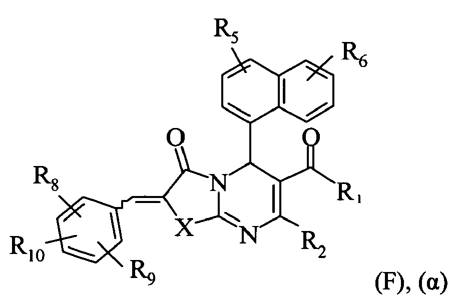 | | | | | | | | | | |

| **No (compound number)** | **X** | **R₁** | **R₂** | **R₈** | **R₉** | **R₁₀** | **R₅** | **R₆** | **Salt** | **Melting point (°C)** |
|---|---|---|---|---|---|---|---|---|---|---|
| 62 (1g) | S | OEt | Me | p-OCOMe | H | H | 4-Me | H | - | >260°C |
| 63 | S | OEt | Me | p-OH | H | o-OH | H | H | - | >260°C |

| **Compounds of formula (Ih)** | | | | | | | | | | |
|---|---|---|---|---|---|---|---|---|---|---|
| 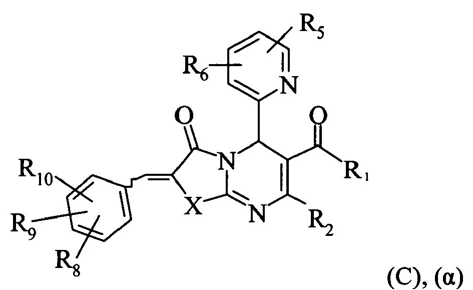 | | | | | | | | | | |

| **No (compound number)** | **X** | **R₁** | **R₂** | **R₈** | **R₉** | **R₁₀** | **R₅** | **R₆** | **Salt** | **Melting point (°C)** |
|---|---|---|---|---|---|---|---|---|---|---|
| 64 (1h) | S | OEt | Me | p-OCOMe | H | H | H | m-(p-OMePh) | - | 91°C |

| **Compound of formula (Ii)** | | | | | | | | | |
|---|---|---|---|---|---|---|---|---|---|
| 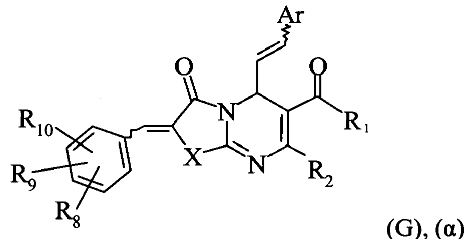 | | | | | | | | | |

| **No (compound number)** | **X** | **R₁** | **R₂** | **R₈** | **R₉** | **R₁₀** | **Ar** | **Salt** | **Melting point (°C)** |
|---|---|---|---|---|---|---|---|---|---|
| 65 | S | OEt | Me | p-OCOMe | H | H | Ph | - | 180 C |

In this table:
- "-" represents a compound in free form,
- OAllyl represents -O-CH₂-CH=CH₂,
- Me represents a methyl group,
- Et represents an ethyl group,
- OMe represents a methoxy group,
- OEt represents an ethoxy group,
- Ph represents a phenyl group,
- o, p, m precises the position of the substitution on the phenyl group,
- n.d. means not determined,

### Example 6: Pharmacological test

The compounds of the invention have been the subject of pharmacological tests which have demonstrated their relevance as active substances against CDC25 and on cancer cell proliferation.

### MATERIAL AND METHODS

### In vitro enzymatic assay

The activity of the MBP-CDC25B3 recombinant enzyme was monitored using fluorescein diphosphate as the substrate. The assay was performed in 96-well plates in a final volume of 200 µL. The maltose binding protein-CDC25B3 was kept in elution buffer [50mM phosphate pH 7.5, 300 mM NaCl, 500 mM imidazole, 10% glycerol]. It was diluted in assay buffer [30 mM Tris-HCl (pH 8.2), 75 mM NaCl, 0.67 mM EDTA, 0.033% BSA, 1 mM DTT]. The final concentration of CDC25B3 was 75 ng/well. Products were studied in a concentration response up to 100 µM. The reaction was initiated by addition of 30 µM of fluorescein diphosphate followed by immediate measure of fluorescein emission with a Fluoroskan Ascent (Lab Systems; excitation filter: 485 nm, emission filter: 530 nm). The results are expressed as the means of at least two independent experiments with three determinations per tested concentration and per experiment. For each compound, the drug concentration required for 50% inhibition (IC₅₀) was determined from a sigmoidal dose-response curve using GraphPad Prism (GraphPad Software, San Diego, CA).

### Clonogenic assay

For cloning inhibition assays, HeLa cells were plated at 100 cells/well in 6-well tissue culture plates with 2.5 mL of medium/well. After 24 h growing at 37 °C in a humidified atmosphere of 5% CO₂, the culture medium was replaced by control medium or medium containing inhibitors at increasing concentrations (up to 100 µM) and incubated for 10 days. Cells were then washed with 1M phosphate-buffered saline and fixed in 10% formaldehyde for 30 minutes at room temperature. They were carefully rinsed with water, stained with 1 mL crystal violet (2 g in 100 mL ethanol, then 2 mL in 100 mL water) for 10 minutes and finally rinsed with water. The plating efficiency was determined by counting the colonies. The results are expressed as the means of two independent experiments with three determinations per tested concentration. For each compound, the IC₅₀ was determined from a sigmoidal dose-response using GraphPad Prism (GraphPad Software, San Diego, CA).

### RESULTS

The compounds of formula (I) were evaluated for *in vitro* enzymatic activity inhibition on the recombinant fused MBP-CDC25B protein with fluorescein diphosphate as the substrate. The tested compounds exhibit anti-CDC25 activity with IC₅₀ values ranking from 1.2 to 100 µM. In particular, compounds (5a), (5b), (1a), (12a) as listed above as well as compounds 1 and 12 of the table show IC₅₀ values of 1.2, 2.9, 6.0, 9.2, 13 and 16.7 µM respectively.

The effects on cell proliferation were further investigated in a clonogenic assay on HeLa cells. HeLa cells were treated at day 1 with increasing concentration of drugs and then plated for an additional 10-day. After crystal violet staining and colony counting, dose-response curve were drawn and the IC₅₀ values determined. Each of the tested compounds was effective at inhibiting clonal proliferation of the Hela cells in a concentration-dependent manner and in particular compound 1, (1a), (5a), (5b) and 12 of the table exhibit IC₅₀ values of 15.9, 14.8, 1.8, 3.5 and 9.75 µM respectively.

Therefore, the results of the tests carried out on the compounds disclosed in the present invention show that, *in vitro,* they exhibit the property to inhibit the enzymatic activity of CDC25B.

For this purpose an effective amount of a said compound may be administered to a subject suffering from a cancer. Among said cancer, the prostate cancer, the colorectal cancer, hepatocarcinomas, head and neck cancer, pancreas cancer, oesophagus cancer, non-hodgkinian lymphomas and thyroid gland cancer may be cited.

A further object of the invention relates to a compound of formula (Ig) with the proviso that at least two of R₈, R₉ and R₁₀ radical are acyloxy groups or with the further proviso that at least one of R₈, R₉ and R₁₀ radical is an acyloxy group with the exception of compounds substituted in the 2-position of the naphtyl group by a methoxy group according to the invention or anyone of the individual compounds (1a) to (17a), (1b) to (8b), (1c), (1g) and (1h), listed above, their salts with pharmaceutically acceptable acids and mixture thereof for use as a medicament.

According to another of its aspects, the present invention relates to pharmaceutical compositions containing an active ingredient, chosen among a compound of formula (Ig) with the proviso that at least two of R₈, R₉ and R₁₀ radical are acyloxy groups or with the further proviso that at least one of R₈, R₉ and R₁₀ radical is an acyloxy group with the exception of compounds substituted in the 2-position of the naphtyl group by a methoxy group or (Ih) according to the invention and anyone of the individual compounds (1a) to (17a), (1b) to (8b), (1c), (1g) and (1h) listed above, their salts with pharmaceutically acceptable acids and mixture thereof.

Thus, these pharmaceutical compositions contain an effective amount of said compound, and one or more pharmaceutical excipients.

The aforementioned excipients are selected according to the dosage form and the desired mode of administration.

In this context the compounds according to the present invention can be present in any pharmaceutical form which is suitable for enteral or parenteral administration, in association with appropriate excipients, for example in the form of plain or coated tablets, hard gelatine and other capsules, suppositories, or drinkable or injectable solutions or suspensions, in doses which enable the daily administration of from 0.1 to 1000 mg of active substance.

The present invention according to another of its aspects, also relates to a method of treatment of cancer which comprises administering to a subject an effective amount of a compound according to formula (I), anyone of compounds of formula (Ia) to (Ii) according to the invention, anyone of the individual compounds (1a) to (17a), (1b) to (8b), (1c), (1g) and (1h) as listed above or one of their pharmaceutically acceptable salts.

## Claims

1. Use of a compound of formula (I) 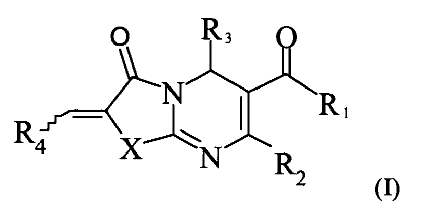 wherein
**R₁** represents a hydroxy group, an amino group, an alkyl group, an aryl group, an alkoxy group, an alkylamino group, an aralkyl group, an arylamino group, an aralkoxy group, an aryloxy group, an aralkylamino group a -O-(CH₂)ₘ-CN group, where m represents 1, 2 or 3,
**R₂** represents an alkyl group, an optionally substituted phenyl group, a trifluoromethyl group,
**X** represents -S- or -NR₁₃-, where **R**₁₃ represents a hydrogen atom, an alkyl group, an alkenyl group, an acyl group, an aralkyl group, an aminoalkyl group, a hydroxyalkyl group, an alkoxycarbonyl group, an aralkoxycarbonyl group or a haloalkyl group,
means that the exocyclic double bond may form a Z or E configuration,
**R₃** represents one of the following groups:
- (A) 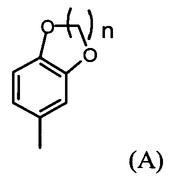 wherein **n** represents 1 or 2,
- (B) an optionally substituted phenyl group 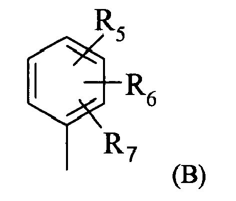 wherein **R₅**, **R₆** and **R₇** independently represent a hydrogen atom, a halogen atom, a hydroxy group, an optionally substituted alkoxy group, an optionally substituted alkyl group, an optionally substituted alkenyl group, an optionally substituted alkenyloxy group, an acyloxy group, an optionally substituted aryloxy group, an optionally substituted phenyl group, a nitro group, an amino group, an alkylaminocarbonyl group, an acylamino group, a carboxy group, a cyano group, a malonyl group, an alkoxycarbonyl group, an acyl group, an amido group, a haloalkyl group, a hydroxyalkyl group, an aminoalkyl group, a haloalkoxy group, an aralkoxycarbonyl group,
- (C) a pyridyl group 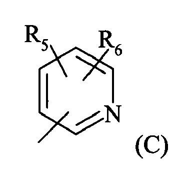 wherein **R₅** and **R₆** are as defined above,
- (D) a quinolinyl group or an isoquinolinyl group 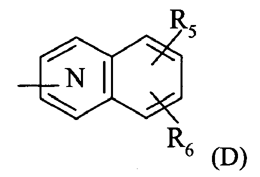 wherein **R₅** and **R₆** are as defined above,
- (E) an indolyl group, 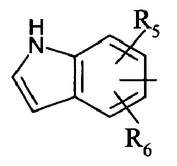 wherein **R₅** and **R₆** groups as defined above,
- (F) a naphthyl group 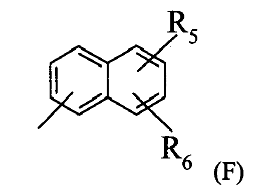 wherein **R₅** and **R₆** are as defined above, and
a 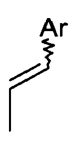 group, where Ar may be an optionally substituted phenyl group,
- (H) a thienyl group 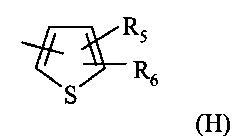 wherein **R₅** and **R₆** are as defined above and,
- (J) a furyl group 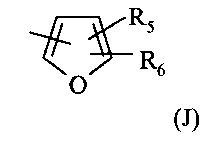 wherein **R₅** and **R₆** are as defined above,
**R₄** represents one of the following groups:
- (α) 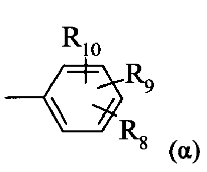 wherein **R₈**, **R₉** and **R₁₀** independently represent a hydrogen atom, a halogen atom, a hydroxy group, an optionally substituted alkoxy group, an optionally substituted alkyl group, an optionally substituted alkenyl group, an optionally substituted alkenyloxy group, an optionally substituted acyloxy group, an optionally substituted aryloxy group, an optionally substituted phenyl group, a nitro group, an amino group, an alkylaminocarbonyl group, an acylamino group, a carboxy group, a cyano group, a malonyl group, an alkoxycarbonyl group, an acyl group, an amido group, a haloalkyl group, a hydroxyalkyl group, an aminoalkyl group, a haloalkoxy group, an aralkoxycarbonyl group,
-(β) 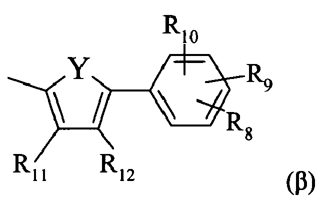 wherein
Y represents -S- or -O-,
**R₈**, **R₉** and **R₁₀** are as defined above, and
**R₁₁** and **R₁₂** independently represent a hydrogen atom or a halogen atom,
- (γ) a pyridyl group 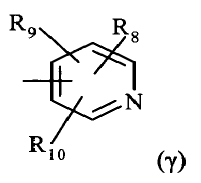 wherein **R₈, R₉** and **R₁₀** are as defined above,
-(δ) 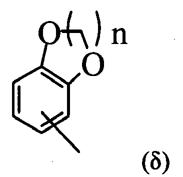 wherein **n** represents 1 or 2,
- (ε)
a 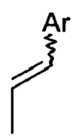 group, where Ar may be an optionally substituted phenyl group,
and wherein the optionally substitution group of the alkyl group, the alkoxy group, the alkenyl group and the alkenyloxy group is one or two groups chosen among a hydroxy group, a halogen atom, a cyano group, a carboxy group and an optionally substituted phenyl group, and
wherein the optionally substitution group of the phenyl group, the aryloxy group and the alkenyl, alkyl, aryl or heteroaryl group of the acyl group may be one, two or three groups chosen among a nitro group, a halogen atom, a methyl group, a hydroxy group, a cyano group, a carboxy group, a methoxy group or an ethoxy group,
for the preparation of a pharmaceutical composition intended for the treatment of cancer.

2. Use according to claim 1, wherein the cancer is chosen among the prostate cancer, hepatocarcinomas, head and neck cancer, oesophagus cancer, thyroid gland cancer and non-hodgkinian lymphomas.

3. Use of a compound of formula (Ia) 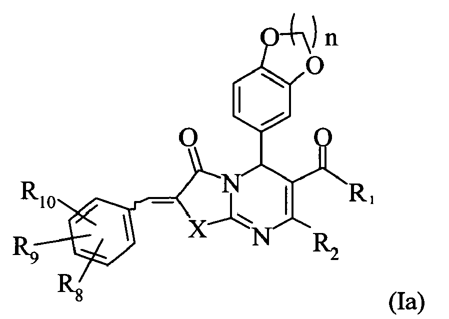 wherein R₁, R₂, R₈, R₉, R₁₀, X and n are as defined in claim 1 for the preparation of a pharmaceutical composition intended for the treatment of cancer.

4. Use of a compound of formula (Ib) 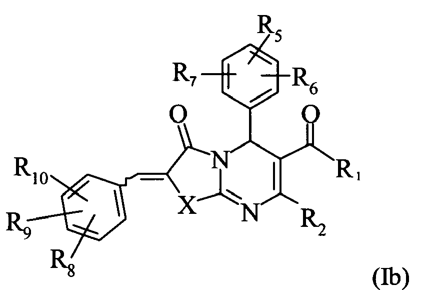 wherein R₁, R₂, R₅, R₆, R₇, R₈, R₉, R₁₀ and X are as defined in claim 1 for the preparation of a pharmaceutical composition intended for the treatment of cancer.

5. Use of a compound of formula (Ic) 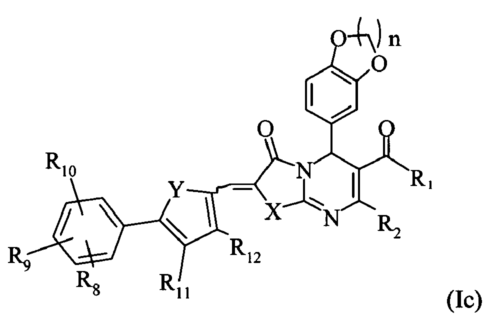 wherein R₁, R₂, R₈, R₉, R₁₀, R₁₁, R₁₂, X, Y and n are as defined in claim 1 for the preparation of a pharmaceutical composition intended for the treatment of cancer chosen among the prostate cancer, hepatocarcinomas, head and neck cancer, oesophagus cancer, thyroid gland cancer and non-hodgkinian lymphomas.

6. Use of a compound of formula (Id) 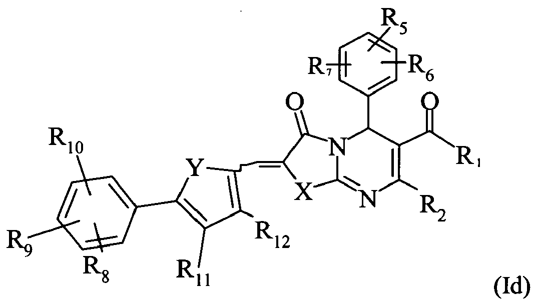 wherein R₁, R₂, R₅, R₆, R₇, R₈, R₉, R₁₀, R₁₁, R₁₂, X and Y are as defined in claim 1 for the preparation of a pharmaceutical composition intended for the treatment of cancer chosen among the prostate cancer, hepatocarcinomas, head and neck cancer, oesophagus cancer, thyroid gland cancer and non-hodgkinian lymphomas.

7. Use of a compound of formula (Ie) 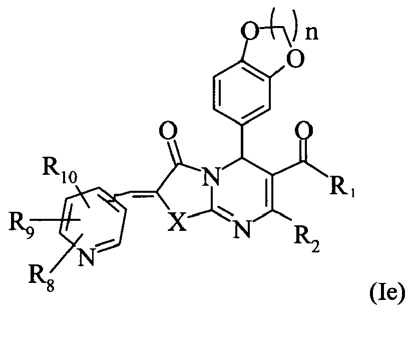 wherein R₁, R₂, R₈, R₉, R₁₀, X and n are as defined in claim 1 for the preparation of pharmaceutical composition intended for the treatment of cancer.

8. Use of a compound of formula (If) 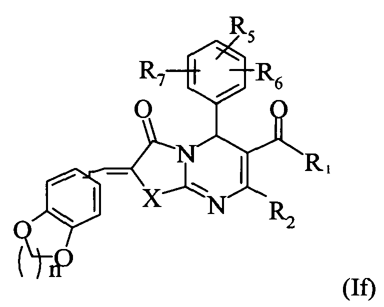 wherein R₁, R₂, R₅, R₆, R₇, X and n are as defined in claim 1 for the preparation of a pharmaceutical composition intended for the treatment of cancer.

9. Use of a compound of formula (Ig) 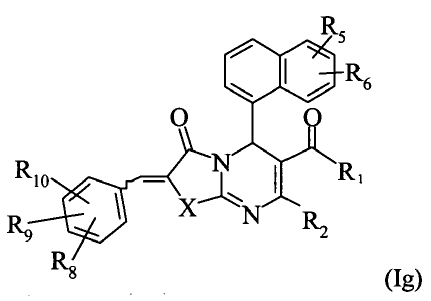 wherein R₁, R₂, R₅, R₆, R₈, R₉, R₁₀ and X are as defined in claim 1 for the preparation of a pharmaceutical composition intended for the treatment of cancer.

10. Use of a compound of formula (Ih) 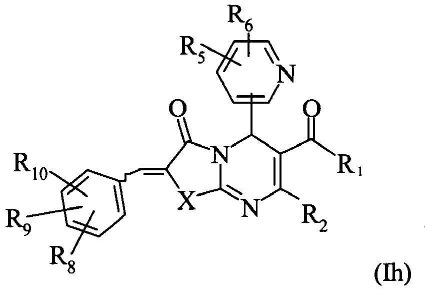 wherein R₁, R₂, R₅, R₆, R₈, R₉, R₁₀ and X are as defined in claim 1 for the preparation of a pharmaceutical composition intended for the treatment of cancer.

11. Use of a compound of formula (Ii) 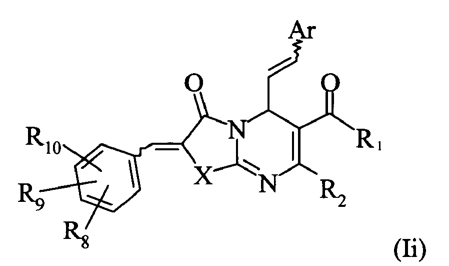 wherein R₁, R₂, Ar, X, R₈, R₉ and R₁₀ are as defined in claim 1 for the preparation of a pharmaceutical composition intended for the treatment of cancer.

12. Compound of formula (Ig) 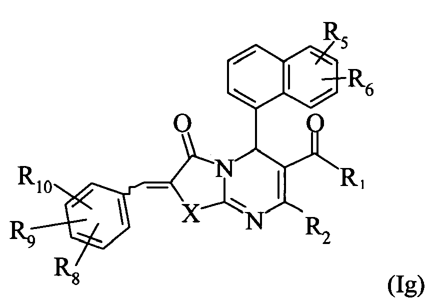 wherein R₁, R₂, R₅, R₆, R₈, R₉, R₁₀ and X are as defined in claim 1, with the proviso that at least two of R₈, R₉ and R₁₀ radical are acyloxy groups or with the further proviso that at least one of R₈, R₉ and R₁₀ radical is an acyloxy group with the exception of compounds substituted in the 2-position of the naphtyl group by a methoxy group, or its pharmaceutically acceptable salt.

13. Compound of formula (Ih) 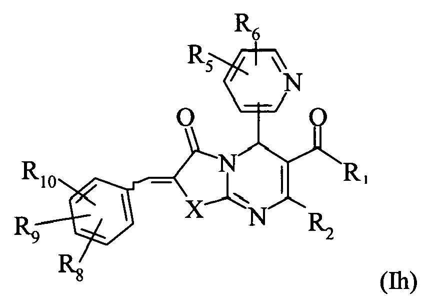 wherein R₁, R₂, R₅, R₆, R₈, R₉, R₁₀ and X are as defined in claim 1 or its pharmaceutically acceptable salt.

14. Compound chosen among
- Compounds of formula (Ia) 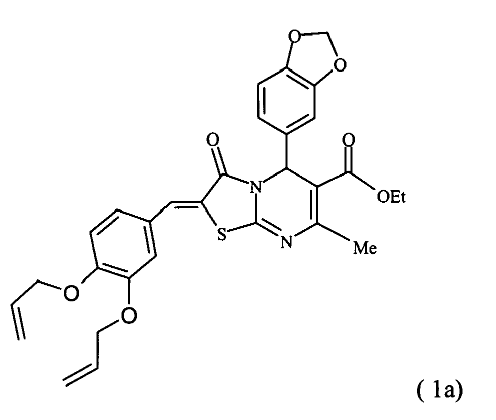 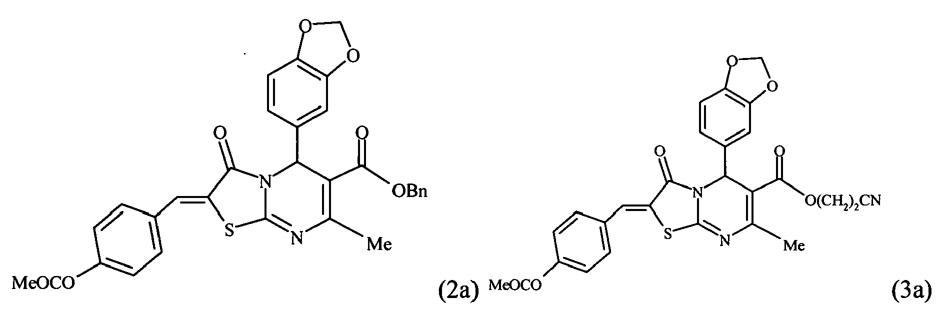 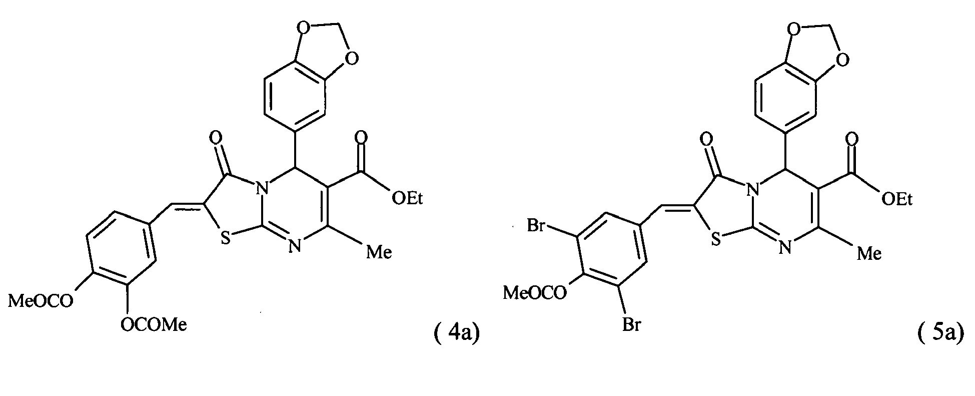 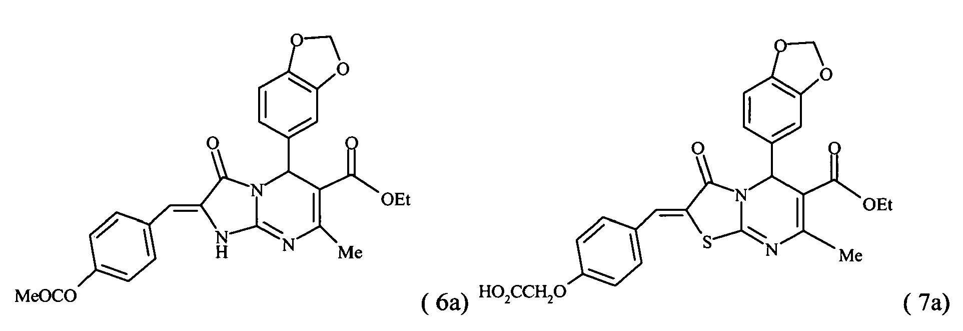 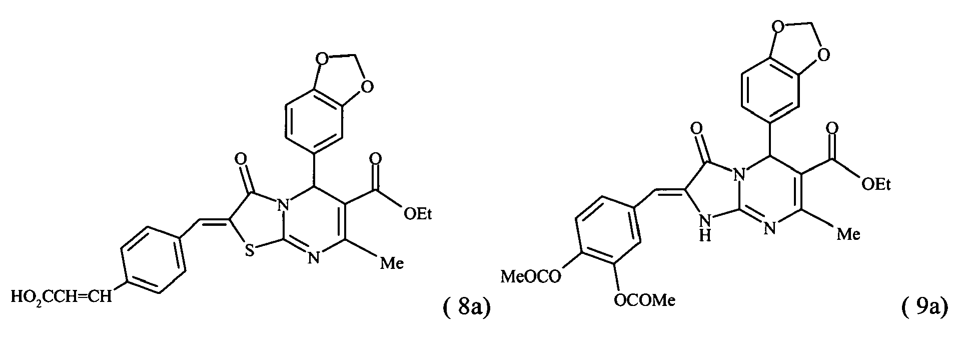 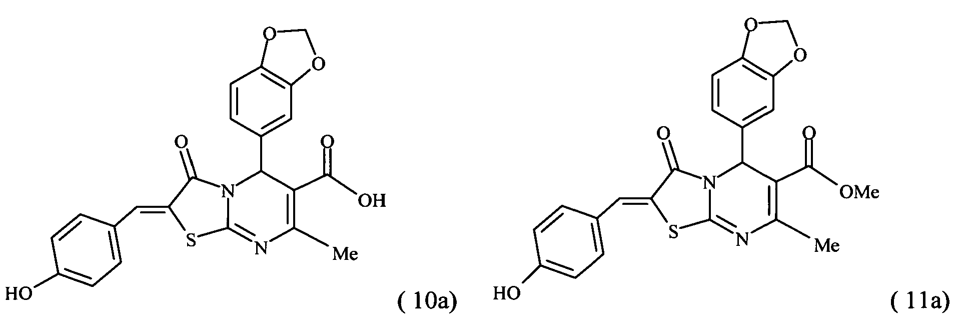 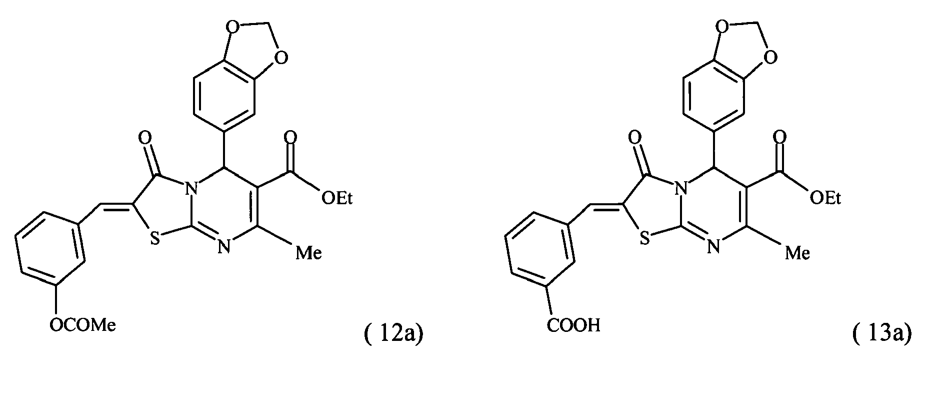 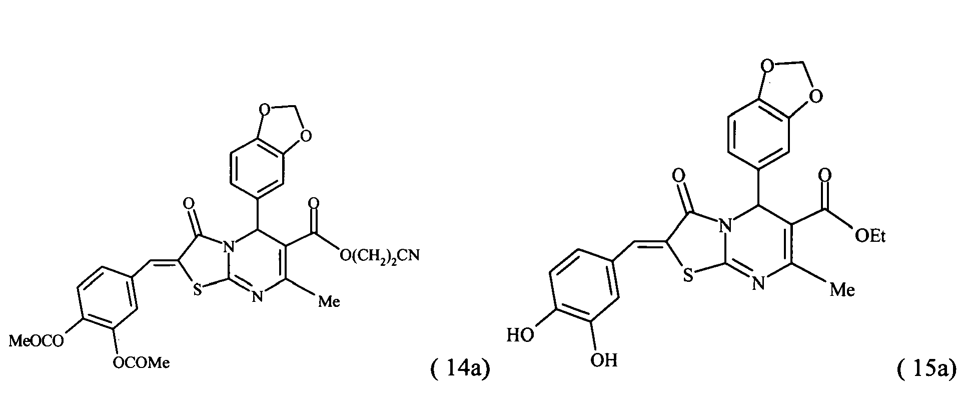 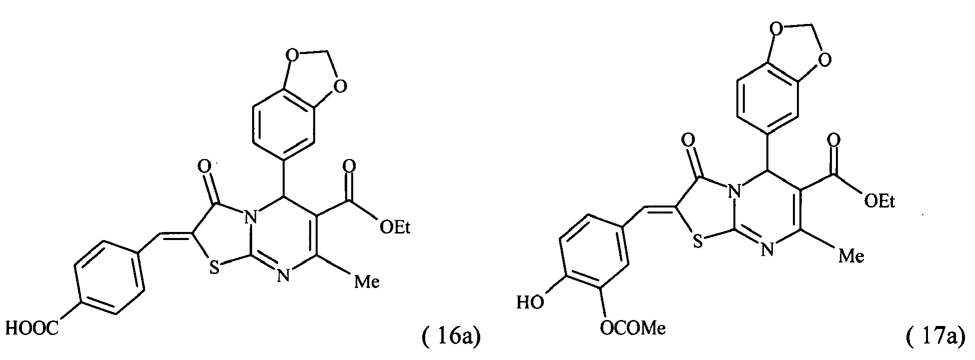
- Compounds of formula (Ib) 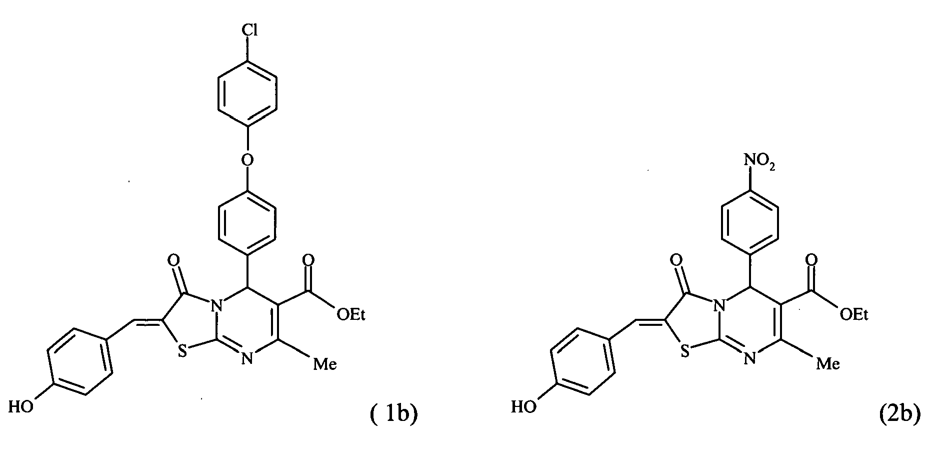 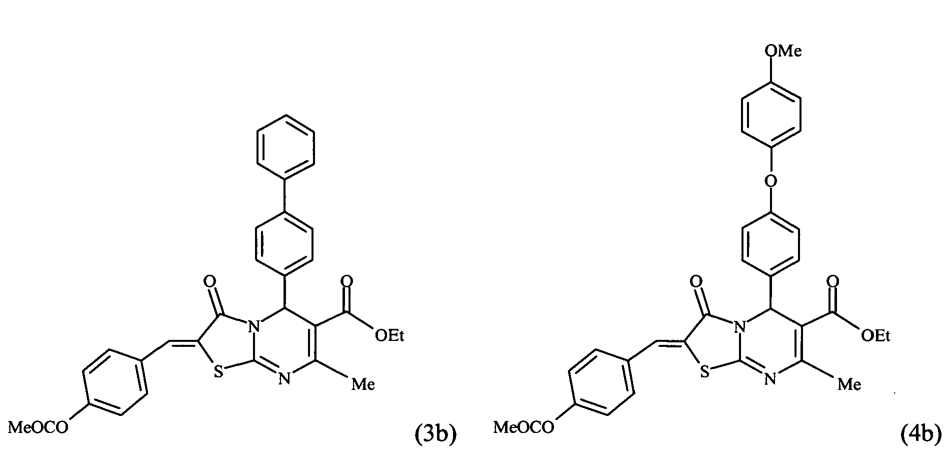 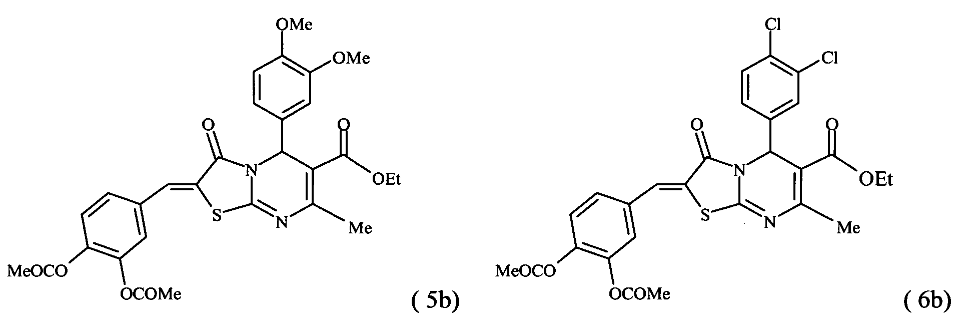 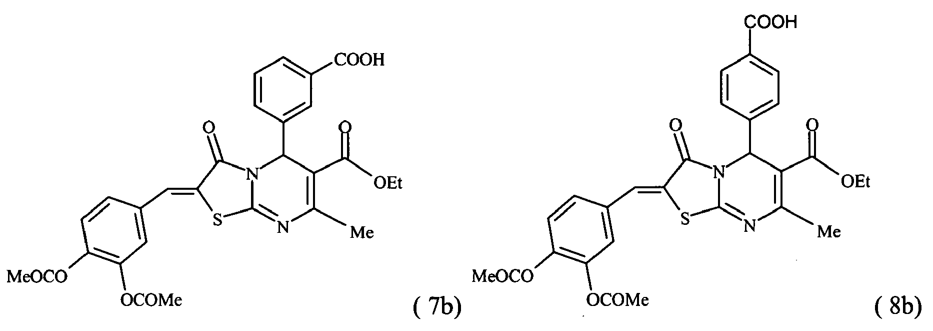
- Compounds of formula (Ic) 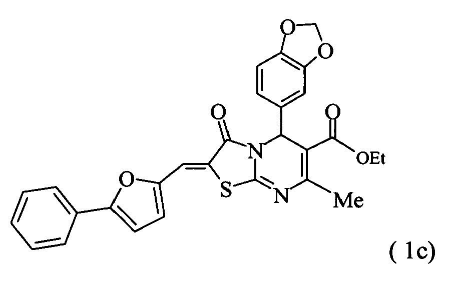
- Compounds of formula (Ig) 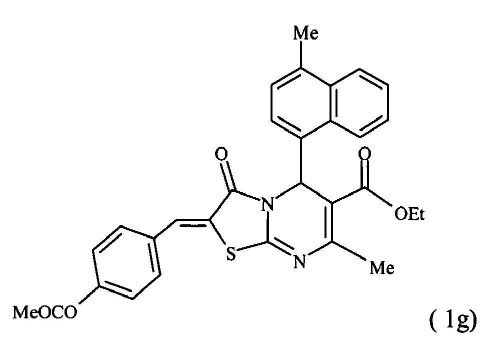
- Compounds of formula (Ih) 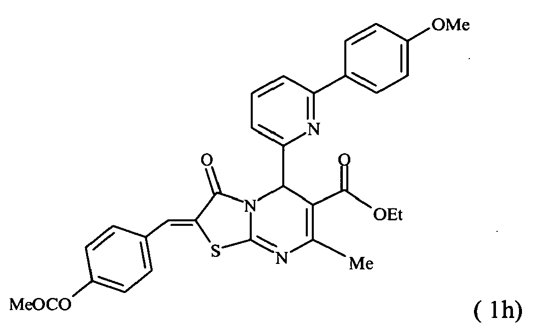
or anyone of their salts with pharmaceutically acceptable acids and mixtures thereof or among the following salified compound.

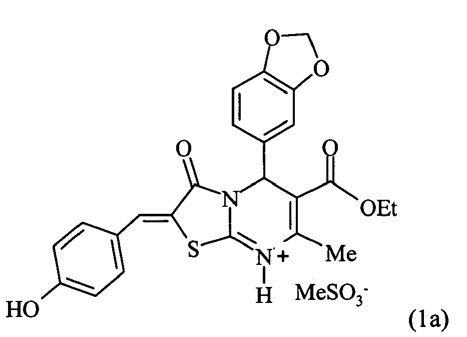

15. Compound according to anyone of claim 12 to 14 for use as a medicament.

16. Pharmaceutical composition containing an active ingredient chosen among a compound of formula (Ig) or (Ih) according to claim 12 or 13, or anyone of compounds (1a) to (17a), (1b) to (8b), (1c), (1g) and (1h) according to claim 14, or mixture thereof.
